# EUROPEAN PATENT SPECIFICATION

(11) **EP 2 294 223 B1**
(45) Date of publication and mention of the grant of the patent: **09.04.2014**
(21) Application number: 09772874.5
(22) Date of filing: 24.06.2009
(51) Int. Cl.: C12Q 1/68

(54) **PROBES FOR USE IN DIAGNOSING PORPHYRIA AND ALLELIC QUANTIFICATION OF PORPHYRIA RELATED GENES BY LIGATION AND AMPLIFICATION REACTIONS**
SONDEN ZUR DIAGNOSE VON PORPHYRIE UND ALLELER QUANTIFIZIERUNG VON PORPHYRIE-ASSOZIIERTEN GENEN MITTELS LIGATIONS- UND AMPLIFIKATIONSREAKTIONEN
SONDES SERVANT À DIAGNOSTIQUER LE PORPHYRIA ET À QUANTIFIER LES ALLÈLES DE GÈNES ASSOCIÉS AU PORPHYRIA AU MOYEN DE RÉACTIONS DE LIGATION ET D'AMPLIFICATION

(30) Priority: 30.06.2008 IT MI20081205
(43) Date of publication of application: 16.03.2011
(73) Proprietor: FONDAZIONE IRCCS ''CA' GRANDA - OSPEDALE MAGGIORE POLICLINICO'', 20122 Milano (IT)
(72) Inventor: DI PIERRO, Elena, I-20068 Peschiera Borromeo (Milano) (IT); CAPPELLINI, Maria Domenica, I-20122 Milano (IT); BESANA, Valeria, I-20040 Cornate d'Adda (Milano) (IT); BRANCALEONI, Valentina, I-20018 Sedriano (Milano) (IT)
(74) Representative: Biggi, Cristina
(86) International application number: PCT/IB2009/006053
(87) International publication number: WO 2010/001212

(56) References cited:
- WO-A-2005/095650
- WHATLEY SHARON D ET AL: "Gene dosage analysis identifies large deletions of the FECH gene in 10% of families with erythropoietic protoporphyria" JOURNAL OF INVESTIGATIVE DERMATOLOGY, vol. 127, no. 12, December 2007 (2007-12), pages 2790-2794, XP002547837 ISSN: 0022-202X
- DI PIERRO E ET AL: "Co-existence of two functional mutations on the same allele of the human ferrochelatase gene in erythropoietic protoporphyria" CLINICAL GENETICS, vol. 71, no. 1, January 2007 (2007-01), pages 84-88, XP002547838 ISSN: 0009-9163
- AURIZI ET AL: "Heterogeneity of mutations in the ferrochelatase gene in Italian patients with erythropoietic protoporphyria" MOLECULAR GENETICS AND METABOLISM, ACADEMIC PRESS, SAN DIEGO, CA, US, vol. 90, no. 4, 30 March 2007 (2007-03-30), pages 402-407, XP022005725 ISSN: 1096-7192
- DI PIERRO ET AL: "A 10376 bp deletion of FECH gene responsible for erythropoietic protoporphyria" BLOOD CELLS, MOLECULES AND DISEASES, LAJOLLA, US, vol. 40, no. 2, 12 February 2008 (2008-02-12), pages 233-236, XP022477805 ISSN: 1079-9796
- TO-FIGUERAS ET AL: "Study of the genotype-phenotype relationship in four cases of congenital erythropoietic porphyria" BLOOD CELLS, MOLECULES AND DISEASES, LAJOLLA, US, vol. 38, no. 3, 4 April 2007 (2007-04-04), pages 242-246, XP022014555 ISSN: 1079-9796
- SELLNER L N ET AL: "MLPA AND MAPH: NEW TECHNIQUES FOR DETECTION OF GENE DELETIONS" HUMAN MUTATION, JOHN WILEY & SONS, INC, US, vol. 23, no. 5, 1 May 2004 (2004-05-01), pages 413-419, XP009058628 ISSN: 1059-7794
- SCHOUTEN JAN P ET AL: "Relative quantification of 40 nucleic acid sequences by multiplex ligation-dependent probe amplification." NUCLEIC ACIDS RESEARCH 15 JUN 2002, vol. 30, no. 12, 15 June 2002 (2002-06-15), page e57, XP002547839 ISSN: 1362-4962
- WHITE STEFAN J ET AL: "Two-color multiplex ligation-dependent probe amplification: Detecting genomic rearrangements in hereditary multiple exostoses" HUMAN MUTATION, vol. 24, no. 1, 2004, pages 86-92, XP002547840 ISSN: 1059-7794
- HARTEVELD C L ET AL: "Nine unknown rearrangements in 16p13.3 and 11p15.4 causing alpha- and beta-thalassaemia characterised by high resolution multiplex ligation-dependent probe amplification." JOURNAL OF MEDICAL GENETICS DEC 2005, vol. 42, no. 12, December 2005 (2005-12), pages 922-931, XP002547841 ISSN: 1468-6244
- DI PIERRO ELENA ET AL: "Multiplex ligation-dependent probe amplification: a novel approach for genetic diagnosis of Porphyria." JOURNAL OF HUMAN GENETICS 2009, vol. 54, no. 8, 2009, pages 479-487, XP002547842 ISSN: 1434-5161

## Description

The present invention relates to probes and to the use of said probes in a diagnostic method and a diagnostic kit for detecting the presence of Porphyria, in particular for detecting the allelic amount of Porphyria-related genes.

The term Porphyria includes a group of seven diseases due to genetic abnormalities, often related to a severe clinical phenotype, deriving from hereditary defect involving at least one of the enzymes of haem biosynthetic pathway, a reactive element of cellular haemoproteins such as haemoglobin, cytochromes, peroxidase etc. The sequences of the seven genes responsible for Porphyria in Homo sapiens are available from NCBI data bank with the following identification numbers(GeneID):
- aminolevulinate delta dehydratase (ALAD) - 210;
- hydroxymethylbilane synthase (HMBS) - 3145;
- uroporphyrinogen synthase (UROS) - 7390;
- uroporphyrinogen decarboxylase (UROD) - 7389;
- coproporphyrinogen oxidase (CPOX) - 1371;
- protoporphyrinogen oxidase (PPOX) - 5498 and
- iron chelatase (FECH) - 2235.

It is known that said seven genes are responsible in Homo sapiens for seven different forms of Porphyria, which can be classified as acute or chronic depending on the clinical symptoms.

Acute Porphyria manifests itself with several symptoms occurring acutely from time to time, induced by hexogen factors such as intercurrent infections, low-calorie diets, intake of alcoholics and drugs with a porphyrinogenic action (barbiturates, sulphonamides, anaesthetics and oral contraceptives), or by endogen factors such as physical and psychic stress and fluctuations of gonadal steroid hormones (estrogens and progestinics) during pre-menstrual periods and pregnancy. Attacks mainly start with neurological symptoms which can show up both in the central and in the vegetative-peripheral nervous system.

Chronic Porphyria, conversely, is characterized by skin photosensitivity related to the accumulation of photoreactive precursors mainly in the epidermis, where they are easily excited by light giving rise to oxygen free radicals with subsequent tissue damage and a strong inflammatory response. Photosensitivity is generally limited to surfaces directly exposed to sun (back of hands, neck, legs and ankles in women), especially if subjected to repeated traumatisms or characterized by an increased skin fragility. However, outer signs of skin damage can often be absent or mainly limited to oedema, erythema and/or urticaria.

The following table shows the relation between gene, Porphyria type and clinical symptoms:

| **Detective gene** | **Porphyria** | **Clinical symptoms** |
|---|---|---|
| HMBS | Acute Intermittent Porphyria (AIP) | Acute Porphyria |
| PPOX | Variegate Porphyria(VP) | Acute Porphyria |
| CPOX | Hereditary Coproporphyria(HCP) | Acute Porphyria |
| ALAD | ALAD Deficiency Porphyria (ADP) | Acute Porphyria |
| UROD | Porphyria Cutanea Tarda (PCT) | Chronic Porphyria |
| FECH | Erythropoietic Protoporphyria (EPP) | Chronic Porphyria |
| UROS | Congenital Erythropoietic Porphyria (CEP) | Chronic Porphyria |

Moreover, it is known that peculiar mutations in the above-mentioned seven genes can involve adjacent genes or flanking intergene regions. The corresponding seven chromosomic regions comprising the involved gene and flanking genes have therefore been selected. Said regions have first been selected so that they included at least one gene upstream and one gene downstream from the involved gene, and then,extended on the basis of the locus gene density. In the framework of the present invention, said chromosomic regions listed below are incorporated in the definition of the gene itself:
gene ALAD: NC_000009:115174000-115213000 (reverse complement)
gene HMBS: NC_000011:118442000-118494000
gene UROS: NC_000010:127443000-127533000 (reverse complement)
gene UROD: NC_000001:45225000-45320000
gene CPOX: NC_000003: 99733000-99863000 (reverse complement)
gene PPOX:NC_000001:159334500-159451000
gene FECH: NC_000018:53290000-53442000 (reverse complement)

The prevalence of Porphyria cannot be established in a definite manner, above all because the diagnosis is often absent. In general, these diseases are believed to have low frequency, the prevalence of the various forms varying from one race to another and from one geographical area to another. In Europe there are about 75,000 patients suffering from acute Porphyria, but a high prevalence can be reached in some geographical areas. Two examples are Scotland, where a patient out of 50,000 suffers from a form of Porphyria, with prevalence of AIP (Intermittent Acute Porphyria) and PCT (Porphyria Cutanea Tarda), and South Africa, where the prevalent form is VP (Variegate Porphyria), with an incidence of one out of 1,000 among the white population.

The diagnosis of Porphyria is not simple today because of the analogy of the clinical picture with other diseases of different origin. Moreover, Porphyria-related diseases are classified as rare and the absence of user-friendly diagnostic tools often causes diagnostic errors.

As a matter of fact, diagnosis today is mainly based on the identification of a high concentration of intermediate products of the biosynthetic pathway (free porphyrines) in erythrocytes, plasma, urines and faeces, which is not always possible in all clinical structures and which is not fully reliable due to physiological fluctuations undergone by these metabolites.

Moreover, the determination of erythrocyte enzymatic activity of the single enzymes can be performed only for some of them. Where possible, such determination is not absolutely reliable due to the overlapping between the range of normal and pathologic values.

Today the identification of genetic abnormalities underlying the different forms of Porphyria makes use of a diagnostic protocol, made up of polymerase chain reaction (PCR) and automatic sequencing, which mainly describes point-like mutations or small deletions-insertions in the coding regions and in the splicing junctions of the genes involved. These types of genetic defects confirm the diagnosis in only 80% of patients with clinical and biochemical symptoms that can be related to Porphyria. In the remaining 20%, which is therefore not diagnosed, recent evidence suggests the presence of chromosomic rearrangements (deletions and duplications) as the cause of Porphyria. Said defects cannot be identified with the standard protocol of analysis.

The identification of these peculiar mutations is performed today with two time-wasting and expensive approaches.

The first approach is the study on "familiar segregation of known polymorphisms". Said approach necessarily requires the patient's biological parents and, for the defect to be identified, the presence of polymorphisms having a significant frequency in the population. Moreover, said approach is not quite decisive since the presence of the aforesaid polymorphisms could not be sufficient to demonstrate the absence of Mendelian segregation, which is absolutely necessary for the diagnosis of gene deletions. However, duplications cannot be identified with said approach.

The second approach is the so-called "gene dosage", a method of relative quantification of amplicones corresponding to the involved gene. Said approach makes use of various pairs of primers with a high risk of dimer formation. Moreover, the different annealing effectiveness of the various pairs of primers negatively affects the amplification reaction, since shorter targets are thermodynamically favoured and their competition reduces the reliability of the relative quantification assay. Eventually, not all involved regions can always be amplified at the same time, so that it might be necessary to perform more than one amplification reaction. PCR conditions of the single fragments can indeed be different and the number of amplicones in Multiplex PCR is anyhow limited.

WHATLEY ET AL, 2007 ("Gene dosage analysis identifies large deletions of the FECH gene in 10% of families with erythropoietic protoporphyria" JOURNAL OF INVESTIGATIVE DERMATOLOGY, vol. 127, no. 12, pages 2790-2794) discloses oligonucleotide primers suitable for quantitative PCR analysis of gene dosage of the FECH gene, associated to Erythropoietic Protoporphyria.

Similar studies of genetic alterations in the FECH gene using oligonucleotides are found in DI PIERRO ET AL, 2007 ("Co-existence of two functional mutations on the same allele of the human ferrochelatase gene in erythropoietic protoporphyria" CLINICAL GENETICS, vol. 71, no. 1, pages 84-88), or AURIZI ET AL, 2007 ("Heterogeneity of mutations in the ferrochelatase gene in Italian patients with erythropoietic protoporphyria" MOLECULAR GENETICS AND METABOLISM, vol. 90, no. 4, pages 402-407).

The Applicant has surprisingly found that the problems mentioned above concerning Porphyria diagnosis can be solved by using specific pairs of probes in ligation reactions, in the presence of at least one enzyme ligase and subsequent ligation-dependent PCR amplification.

Ligation-dependent PCR amplification reactions are known in the art as MLPA (Multiplex ligation dependent probe amplification), in particular when several reactions are performed. The pairs of probes include each a different nucleotide sequence complementary to human antisense sequence (hybridising sequence) and, on the said sequence termini a fixed non-human nucleotide sequence (tag). The amplification by PCR reaction occurs only if the pair of probes recognises the wild-type target sequence and the two probes locate one adjacent to the other so as to be linked. The presence of ligase allows probes linkage to make unique sequence to be amplified by PCR using primers coupling with tag sequences.

Depending on the structure of the original probes and on the result of the PCR reaction amplification, the skilled technician is able to understand how a nucleic acid sequence, e.g. a gene, can show different results with respect to the wild-type sequence.

The result of PCR reaction enables a relative quantification of genes involved in Porphyria, with high sensitivity and reproducibility.

The present invention is disclosed in the following detailed description and further below thanks to the accompanying figures.

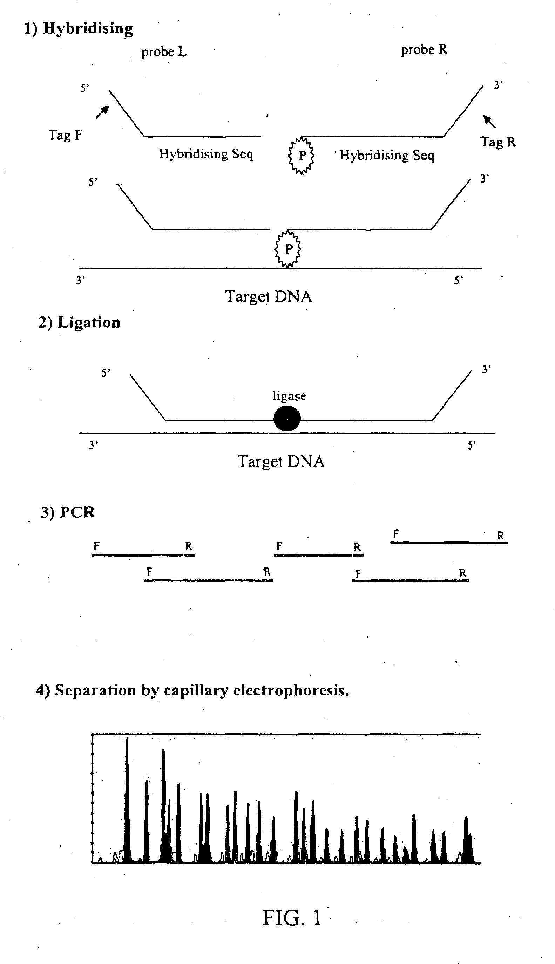

Figure 1 schematically shows specific pairs of probes according to the invention (probe L and probe R in which 5'end is phosphorylated) and how these hybridise to the specific DNA sequence (target DNA) (1); their ligation in the presence of target DNA (2); amplified products deriving from PCR reaction (3), and how capillary electrophoresis enables to obtain results with a diagnostic significance (4) (see Example 1 for further information). As can be observed, the tags present in the probes, referred to as tag F and tag R, are still present in the amplified products resulting from the amplification of the linked probe.

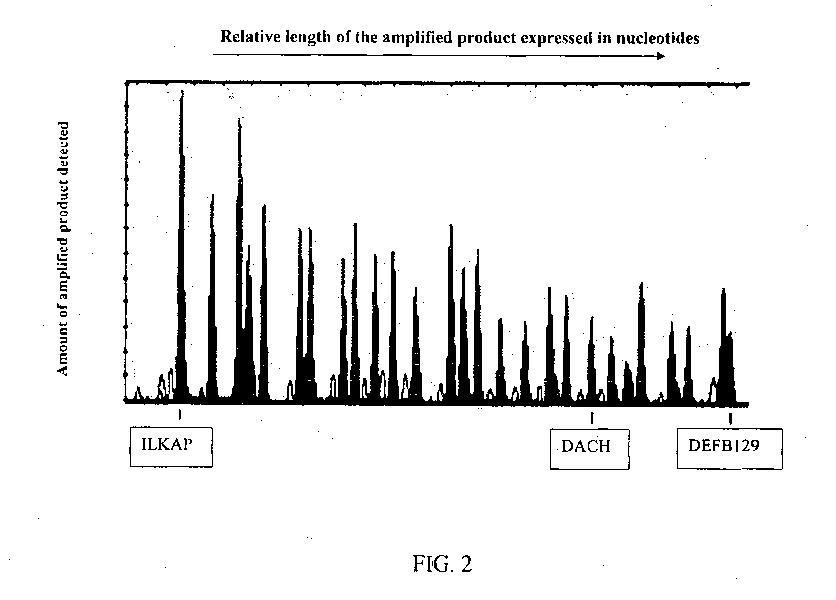

Figure 2 shows an example of peaks obtained by capillary electrophoresis of the amplification products of PCR reaction (amplified products) on DNA from a sample according to the protocol described in Example 1. Note that there are 27 peaks and 3 out of 27 peaks represent inner control probes used to normalize the results of other peaks. These peaks are referred to with the name of the gene to which the inner control probes correspond.

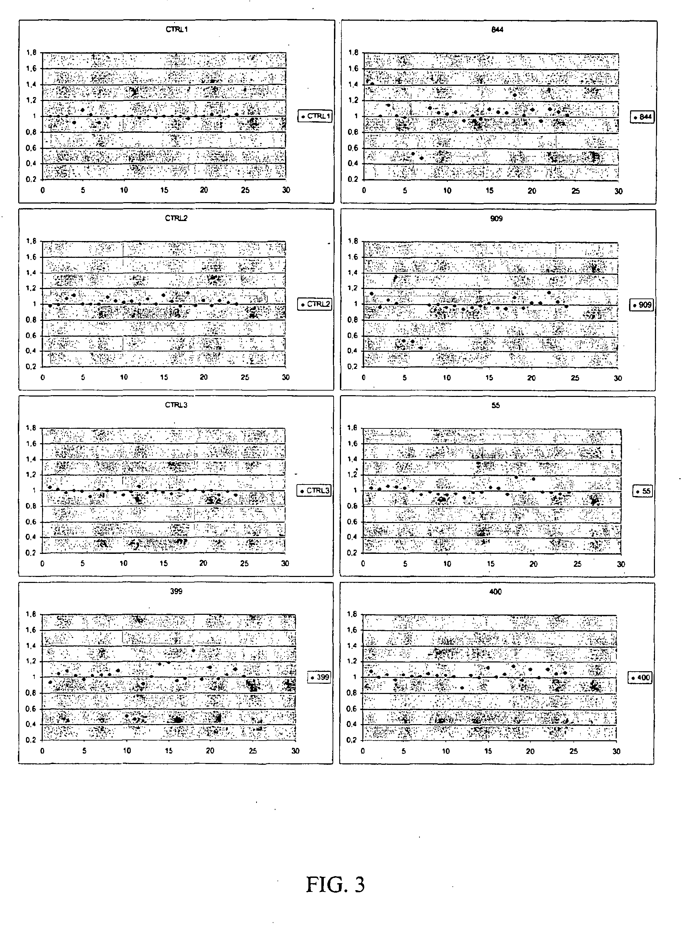

Figure 3 shows the results of the diagnostic method for detecting the state of gene FECH, schematically and after normalization with three inner controls, of various patients (see Example 1). CTRL 1, 2 and 3 represent some healthy subjects on whom the other results have been calibrated (outer control). Note that patients 55 and 400 show no abnormalities with respect to controls, whereas patients 399, 744 and 909 show abnormalities in gene FECH.

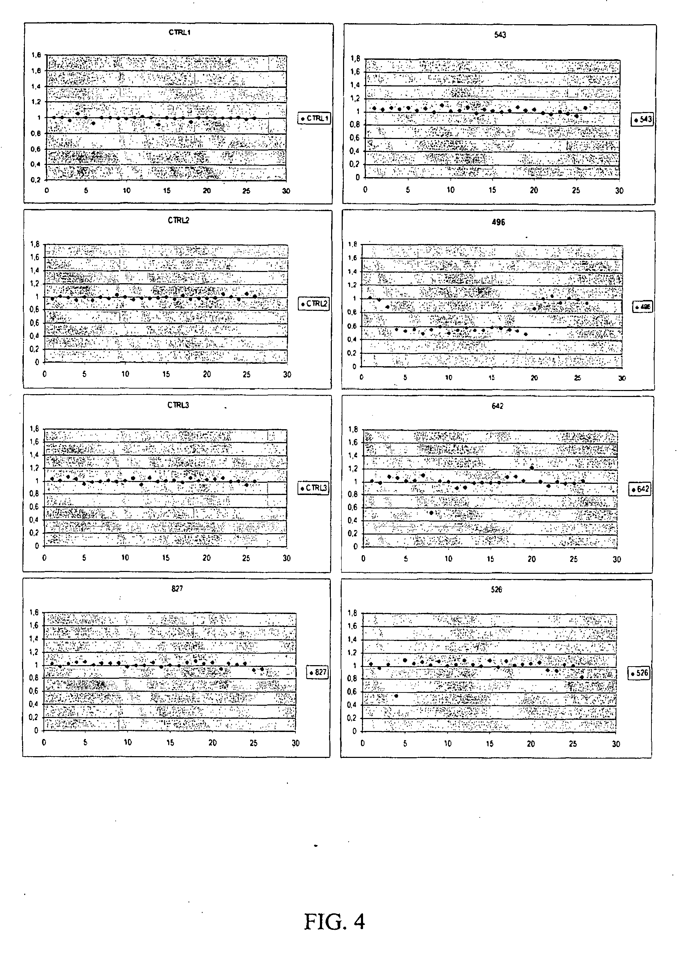

Figure 4 shows the results of the diagnostic method for detecting the state of gene HMBS, schematically and after normalization with the three inner controls, of various patients (see Example 2). CTRL 1, 2 and 3 represent some healthy subjects on whom the other results have been calibrated (outer control). Patients 543 and 827 show no abnormalities with respect to controls, whereas patients 496, 526 and 642 show abnormalities in gene HMBS.

A first object of the present invention is therefore an association of probes comprising a group of probes having at one end of the sequence a tag and at the other end a sequence hybridising to at least one part of the sequence of the FECH gene said hybridising sequences being SEQ ID NO: 1-24 and a group of probes having at one end of the sequence a tag and at the other end a sequence hybridising to at least one part of the sequence of the FECH gene said hybridising sequences being SEQ ID NO: 142-165.

A second object is a method for determining the presence of Erythropoietic Protoporphyria in a sample of biological tissue including the following steps:
(i) extracting nucleic acid from a sample;
(ii) introducing the association of probes into the nucleic acid extracted according to step (i), and reacting in the presence of at least one enzyme ligase in at least one ligation reaction;
(iii) performing at least one amplification reaction on the probes obtained from (ii); and
(iv) determining the presence and amount of nucleotide sequences resulting from ligation and amplification.

A third object is a kit for diagnosing Erythropoietic Protoporphyria including at least one compartment containing the reagents for performing at least one ligation and at least one PCR reaction, and at least one compartment containing the association of probes. cleotide sequences resulting from ligation and amplification.

The nucleic acid extracted in step (i) is preferably DNA.

The skilled technician can select a suitable method among the methods known in the art for extracting nucleic acid from the sample. Preferably, a commercially available kit can be used, such as e.g. Puregene of Qiagen.

In the method according to the disclosure the sample used in step (i) can be taken from any kind of human biological tissue. Preferably, the human biological tissue is peripheral blood.

The pairs of probes referred to in step (ii) have each a sequence hybridising specifically to a target nucleotide sequence and a tag F or tag R on the ends of said probe (on 5' or 3'ends, respectively; see Figure 1) .

Said hybridising sequences are chosen so that the pairs of probes locate adjacent to one another on a part of the sequences of the seven genes of haeme biosynthetic pathway so as to be linked by a ligase, as shown in Figure 1.

The pairs can be designed by the skilled technician by selecting a suitable methods among the methods known in the art for designing specific nucleotide sequences. Preferably, the probe of the pair in position 3' is phosphorylated. More preferably, the probes according to the disclosure have the following properties:
- melting temperature of the hybridising sequence higher than or equal to 67.5°C and
- the content of GC pairs (pair of guanine-cytosine bases) of hybridising sequence is 30 to 63% and
- deltaG (folding thermodynamics) of the whole probe higher than or equal to zero at a temperature of 60°C with a Na⁺ concentration of 0.35M and
- at least one C or G adjacent to the tag (5' and 3', respectively) and
- max. 4 G and/or C bases adjacent to the tag.

Preferably, the hybridising sequences are chosen among sequences comprising SEQ ID Nos. 1-282.

In the framework of the present invention, the sequences are designated according to WIPO International Standard ST.25 and their description has been developed with Patent-In 3.5 software. The description of the sequences mentioned in the present invention is attached hereto.

The probes are preferably DNA.

The skilled technician can suitably choose among reagents, methods, protocols and tags known in the art for performing and inferring data from an amplification reaction known in the art as MLPA and as disclosed above.

The probes according to the disclosure can preferably be divided into two groups:
1. Probes L, in which the probe has a tag on 5'end of the nucleotide sequence and on the other end a hybridising nucleotide sequence as described above and as shown in Figure 1. Preferably, the hybridising sequence of probe L has maximum two nucleotides GC on 3'end. More preferably, the hybridising sequence is chosen among SEQ ID NO. 1 to 141. Preferably, probes L consist of a sequence selected among SEQ ID NO. 1 to 141 and a tag F on 5'end of said sequence. Preferably tag F for probes L is SEQ ID NO 283.
2. Probes R, in which the probe has a tag R on 3'end of the nucleotide sequence and on the other end a hybridising nucleotide sequence as described above, phosphorylated on 5'end and in position 3' with respect to probe L and so as to be linked by a ligase, as shown in Figure 1. Preferably, the hybridising sequence in position 3' with respect to probe L so as to be linked by a ligase is chosen among SEQ ID NO. 142 to 282. Preferably, probes R consist of a sequence selected among SEQ ID NO. 142 to 282 and a tag R on 3'end of said sequence. Preferably, the tag for probes R is SEQ ID NO 284.

Preferably, the sequences of probes L and R consist of SEQ ID NOs. 1-282 and tags SEQ ID NOs. 283-284 as referred to above, because it is thus possible to minimize the total length of the probes L and R, respectively. A minimized length of the probes enables a chemical synthesis thereof, instead of using more expensive methods, without compromising the suitability of said probes in the method according to the invention.

In the step (ii) of the method according to the disclosure, the probes are present in at least one specific pair. Said at least one specific pair is a pair of at least one probe L and at least one probe R as referred to above. Preferably, probe L shows the hybridising sequence SEQ ID NO. n on 3'end of the nucleotide sequence, wherein n is an integer ranging from 1 to 141, and probe R shows the hybridising sequence SEQ ID NO. m on 5'end of the nucleotide sequence, wherein m is n+141.

In step (iii), the skilled technician can suitably choose among all protocols and methods known in the art for performing the at least one amplification. Preferably, at least one amplification is at least one PCR reaction, more preferably according to a MLPA protocol. Preferably, in the at least one PCR reaction, F and R primers are introduced coupling with all F and R tags, respectively, present in the probes added in step (ii). In particular, primer F is the forward primer (i.e. corresponding to tag F), whereas primer R is the reverse primer (therefore reverse complementary to tag R).

Step (iv) includes the determination of the presence and relative amount of nucleic acid resulting from ligation and amplification obtained in steps (ii) and (iii). The presence can be determined with methods known in the art for dividing sequences of nucleic acid depending on their length, such as e.g. by a protocol of capillary electrophoresis. The skilled technician, knowing the length of the pair of initial probes and their hybridising position on DNA, is able to calculate the length of the resulting amplified product. Said skilled technician can then check if the amplified product is present or not such length. The reduction or the increase of the resulting amplified product indicates the occurrence of an abnormality in the nucleic acid extracted in step (i) in the position in which one of the probes responsible for said amplified product in both alleles hybridises.

In a preferred embodiment of the method according to the invention as described above, primer F or primer R is suitably marked on one end. Preferably, the marked primer is primer F marked on 5'end. The marked primer remains suitable for the method according to the invention and enables in step (iv) to recognise the sequence by means of the marker. The marker can be suitably selected among markers known in the art, such as e.g. radioactive atoms, fluorochromes or further marked antibodies, also depending on the determination method used in step (iv), on the presence and amount of sequences of nucleic acids resulting from ligation and amplification. In a preferred embodiment, the marker linked to the primer is a fluorochrome, such as e.g. FAM (6-carboxy fluorescein) or HEX. In said embodiment, the presence and amount of the amplified products resulting from step (iii) can be measured by capillary electrophoresis with a commercially available sequencer, such as e.g. Abi Prism 310. The resulting peaks relate to the presence and amount of the resulting amplified products.

Examples 1 and 2 describe in detail exemplary and absolutely non-limiting embodiments of the method according to the invention.

The amount of the resulting amplified product can be the same or approximately half or approximately twice as much with respect to other amplified products resulting from the same reaction and/or with respect to an amplified product resulting from the use of the same probes on wild-type DNA (outer control). If the amount of amplified product is approximately half, it is believed that there is an abnormality in a copy of the gene, in particular an abnormality in one of the alleles and not in the other one. If the amount of amplified product is approximately twice as much, it is believed that there is a genotype abnormality characterized by a duplication mutation of both gene copies or by a triplication mutation of one gene copy only. In a preferred embodiment of the method according to the invention, at least one pair of probes for inner control is introduced in step (ii), preferably together with said at least one pair of probes. The pairs of probes for inner control are prepared in the same way as for probes L and R according to the invention as described above, the only difference being that said pairs for inner control have hybridising sequences for portions of genes which are known as not involved in human genetic diseases. Preferably, the hybridising sequences in the probes for inner control are selected among pairs SEQ ID NOs. 285-286, 287-288 and 289-290. The use of said probes for inner control is advantageous since it allows to determine whether PCR reaction occurred correctly and/or it enables to normalize the amount of an amplified product, representing the diploid amount (2n). Pair SEQ ID NO. 285-286 corresponds to part of the gene ILKAP, which encodes for a phosphatase known to have a function in the regulation of intra- and/or inter-cellular signals. Pair SEQ ID NO. 287-288 corresponds to part of the gene DACH, which encodes for a protein known to play a role in the development of several organisms. Pair SEQ ID NO. 289-290 corresponds to part of the gene DEFB129, which encodes for a protein known to play a role in the immune system. It is still more advantageous to use all the three control pairs since the three amplified products are of different lengths and enable to normalize amplified products of different lengths. Amplified products resulting from pairs of probes containing the hybridising sequences SEQ ID NOs. 285-286, 287-288 and 289-290 have a length of 132 nucleotide, 118 nucleotides and 80 nucleotides, respectively.

In a preferred embodiment of the method according to the disclosure, said at least one ligation and amplification reaction of steps (ii) and (iii) is selected among seven possible reactions, wherein in each reaction at least one specific pair of probes reacts with one of the seven genes of haeme biosynthetic pathway responsible for Porphyria.

Preferably, the specific pairs of probes in each of the seven possible reactions can be selected from the following groups for the ligation-PCR reactions as follows:
- Ligation-PCR reaction 1 = pair of probes in which a probe has the hybridising sequence ID NO. n wherein n is an integer ranging from 1 to 24, and the other probe has the hybridising sequence SEQ ID NO. m wherein m is n+141. The absence or presence in halved amounts of amplified products resulting from said probes corresponds to a resulting abnormality for the gene FECH.
- Ligation-PCR reaction 2 = pair of probes in which a probe has the hybridising sequence ID NO. n wherein n is an integer ranging from 25 to 47, and the other probe has the hybridising sequence SEQ ID NO. m wherein m is n+141. The absence or presence in halved amounts of amplified products resulting from said probes corresponds to a resulting abnormality for the gene HMBS.

- Ligation-PCR reaction 3 = pair of probes in which a probe has the hybridising sequence ID NO. n wherein n is an integer chosen among 48-71, and the other probe has the hybridising sequence SEQ ID NO. m wherein m is n+141. The absence or presence in halved amounts of amplified products resulting from said probes corresponds to a resulting abnormality for the gene PPOX.
- Ligation-PCR reaction 4 = pair of probes in which a probe has the hybridising sequence ID NO. n wherein n is an integer ranging from 72 to 87, and the other probe has the hybridising sequence SEQ ID NO. m wherein m is n+141. The absence or presence in halved amounts of amplified products resulting from said probes corresponds to a resulting abnormality for the gene CPOX.
- Ligation-PCR reaction 5 = pair of probes in which a probe has the hybridising sequence ID NO. n wherein n is an integer ranging from 88 to 105, and the other probe has the hybridising sequence SEQ ID NO. m wherein m is n+141. The absence or presence in halved amounts of amplified products resulting from said probes corresponds to a resulting abnormality for the gene UROD.

- Ligation-PCR reaction 6 = pair of probes in which a probe has the hybridising sequence ID NO. n wherein n is an integer ranging from 106 to 125, and the other probe has the hybridising sequence SEQ ID NO. m wherein m is n+141. The absence or presence in halved amounts of amplified products resulting from said probes corresponds to a resulting abnormality for the gene UROS.
- Ligation-PCR reaction 7 = pair of probes in which a probe has the hybridising sequence ID NO. n wherein n is an integer chosen among 126-141, and the other probe has the hybridising sequence SEQ ID NO. m wherein m is n+141. The absence or presence in halved amounts of amplified products resulting from said probes corresponds to a resulting abnormality for the gene ALAD.

As a consequence, the choice of which reactions, and in which sequence, have to be performed in order to attain a precise diagnosis is substantially based on results from biochemical tests and/or on the clinical study previously carried out on the patient.

In a still more preferred embodiment, the seven possible ligation-PCR reactions, as listed above, include probe associations comprising all hybridising sequences SEQ ID NOs. 1-282 divided into seven separate associations for the seven ligation-PCR reactions following the above distribution. Therefore, the association for ligation-PCR Reaction 1 comprises a group of probes L having the hybridising sequences SEQ ID NO. 1 to 24 and a group of probes R having the hybridising sequences SEQ ID NO. 142 to 165,
the association for ligation-PCR Reaction 2 comprises a group of probes L having the hybridising sequences SEQ ID NO. 25 to 47 and a group of probes R having the hybridising sequences SEQ ID NO. 166 to 188,
the association for ligation-PCR Reaction 3 comprises a group of probes L having the hybridising sequences SEQ ID NO. 48 to 71 and a group of probes R having the hybridising sequences SEQ ID NO. 189 to 212,
the association for ligation-PCR Reaction 4 comprises a group of probes L having the hybridising sequences SEQ ID NO. 72 to 87 and a group of probes R having the hybridising sequences SEQ ID NO. 213 to 228,
the association for ligation-PCR Reaction 5 comprises a group of probes L having the hybridising sequences SEQ ID NO. 88 to 105 and a group of probes R having the hybridising sequences SEQ ID NO. 229 to 246,
the association for ligation-PCR Reaction 6 comprises a group of probes L having the hybridising sequences SEQ ID NO. 106 to 125 and a group of probes R having the hybridising sequences SEQ ID NO. 247 to 266, and
the association for ligation-PCR Reaction 7 comprises a group of probes L having the hybridising sequences SEQ ID NO. 126 to 141 and a group of probes R having the hybridising sequences SEQ ID NO. 267 to 282.

More preferably, the nucleotide sequences of the probes consist of a sequence of nucleic acid chosen among sequences SEQ ID Nos. 1-282 as just described above, and a tag SEQ ID Nos. 283 or 284 if the probes are L or R, respectively.

The advantage of using said associations of probes is that each sequence resulting from amplification varies in total length of 80-132 nucleotides and differs in length from other sequences in the same association (i.e. for the same PCR reaction) of at least 2 nucleotides. This advantageously enables to use up to 27 pairs of probes with the same marker in a single ligation-PCR reaction and to be able to discriminate among the results for all the 27 pairs of probes (corresponding to a specific area of a gene).

From the results obtained in step (iv) the skilled technician can infer, with a precision of 80-132 nucleotides, the location of an abnormality or an allelic difference in one or more of the seven genes involved in Porphyria aetiology.

If more ligation-PCR reactions are to be performed, these can be carried out in separate test tubes, either simultaneously or sequentially.

In order to reduce ligation-PCR reactions, before starting the method described above it is assessed whether the patient suffers from gene abnormalities for acute or chronic Porphyria with the methods known in the art. If the patient shows gene abnormalities for acute Porphyria, Associations for ligation-PCR Reaction 2, 3, 4 and 7 are used; whereas if the patient shows gene abnormalities for chronic Porphyria, Associations for ligation-PCR Reaction 1, 5 and 6 are used. Based on the analysis of the clinical and biochemical occurrences of the Porphyria type the patient suffers from, in some cases the method can be limited to the use of one association only, related to the type of Porphyria disease to be diagnosed.

In another embodiment of the method according to the invention, two or more associations of probes for ligation-PCR reactions are used, selected from 1-7 as described above, in a single ligation-PCR reaction by using different tags F and R in the probes belonging to the two or more associations of probes. The different tags used are specific for one of the two or more associations and different markers are linked to the group of primers present in PCR reaction of step (iii). By using specific markers associated to primers coupling the different tags F and R, it can be discriminated between the different associations, or probes, thus obtaining more results from a single ligation-PCR reaction in step (ii) and (iii).

Another object of the disclosure includes probes having at one end of the sequences a tag and at the other end a sequence hybridising to at least part of the sequences of the seven genes of haeme biosynthetic pathway involved in Porphyria aetiology. Preferably, the probes are provided with the properties in their sequences as mentioned above. Preferably, the hybridising sequence is chosen among SEQ ID NOs. 1-282. More preferably, the tag sequence is chosen between SEQ ID NOs. 283 and 284.

A further object of the present disclosure includes associations of probes having nucleotide sequences as disclosed above.

Reference is made to the appended claims for preferred embodiments.

Another object of the present disclosure is a diagnostic kit comprising at least one compartment containing the reagents for performing the at least one ligation of step (ii) and the at least one amplification of step (iii), and at least one compartment containing the probes to be used for at least one of the seven associations for ligation-PCR reactions 1-7, as already described above. Preferably, there are seven compartments containing the probes, each of them including one of the seven associations for ligation-PCR reactions 1-7 as described above. Preferably, the diagnostic kit comprises at least one pair of probes for inner control, preferably probes including SEQ ID NOs. 285-290 as already described above.

### Example 1 - Diagnosis of the presence of Porphyria-related abnormalities in gene FECH

An analysis was performed on 30 subjects, 15 healthy and 15 with clinical symptoms which can be related to Porphyria. A sample of 7 ml of peripheral blood in EDTA was taken from each person. The sample was centrifuged at 3,000 rpm for 10 minutes at 4°C. After removing plasma, a physiologic solution was added and the whole was mixed. Then it was centrifuged at 3,000 rpm for 10 minutes at 4°C and after removing the physiological solution, 150 µl of buffy coat (lymphomonocytes) were taken. DNA was extracted with the kit Puregene - Gentra (QIAGEN) adding to the sample 450 µl of RBC Lysis Solution 1 and stirring for 10 minutes at room temperature (RT). Then it was centrifuged at 14,000 rpm for 1 minute at RT, the supernatant was removed and the pellet re-suspended. 450 µl of Cell Lysis Solution 2 were added and the pellet was re-suspended. 150 µl of Protein Precipitation Solution 3 were added and it was vortexed for 20 seconds. The supernatant was recovered after centrifugation at 14,000 rpm for 3 minutes at RT. 100% v/v isopropanol was added. It was centrifuged at 14,000 rpm for 1 minute and after removing the supernatant, 300 µl of 70% ethanol were added. It was centrifuged at 14,000 rpm for 1 minute at RT and after removing the supernatant and drying the pellet with Speed Vacuum for 2 minutes, the pellet was re-suspended in 100 µl of TE (Tris-HCl 10mM and EDTA 1mM). The DNA was then diluted at a final concentration of 20 ng/µl in TE.

The sequences of the probes and of the primers were obtained by chemical synthesis.

The mix of pairs of probes for the gene FECH was prepared by joining 2.4 µl of each probe (probes L: SEQ ID NO. 283+ SEQ ID NOs. 1-24 and probes R: SEQ ID NOs. 142-165+ SEQ ID NO. 284) with a concentration of 1 pmol/µl. (FECH probe mix). The 3 pairs of probes of the control genes was also added to the mix(probes L: SEQ ID NO. 283+ SEQ ID NOs. 285, 287, 289 and probes R: SEQ ID NOs. 286, 288, 290+ SEQ ID NO. 284) in a total final volume of 600 µl of TE.

The primers F (SEQ ID NO. 283 marked with fluorochrome FAM at 5'end) and R (SEQ ID NO. 291) were diluted at a concentration of 20 pmol/µl in TE and a 10 mM concentrated aliquote of dNTPs was prepared.

The test made use of the kit MLPA reagents 100 reactions kit (MRC-Holland-RESNOVA) except for SALSA PCR primer mix.

100 ng of DNA was denatured at 98°C for 5 minutes; 2 µl of a mixture comprising 1.5 µl of FECH probe mix and 1.5 µl of SALSA MLPA buffer at 25°C was added. The whole was incubated for 1 minute at 95°C and for at least 16 h at 60°C (hybridising). A mixture containing 3 µl of buffer A, 3 µl of ligation buffer B, 25 µl of sterile water and 1 µl of ligase was prepared and added to DNA at 54°C. Then it was incubated at 54°C for 15 minutes, 98°C for 5 minutes and stored at 4°C (ligation). 15 µl of mix 1 of PCR, containing 13 µl of water and 2 µl of SALSA PCR buffer were aliquoted in sterile tubes. 5 µl of ligated product were added and with the pipes at 60°C 5 µl of mix 2 of PCR were added, containing 2.75 µl of water, 1 µl of Salsa Enzyme dilution buffer, 0.5 µl of dNTPs, 0.25 µl of SEQ ID NO 283 and 0.25 µl of SEQ ID NO 291 and 0.25 µl of SALSA Polymerase. Then the 35-cycle PCR protocol was started: 30 sec at 95°C; 30 sec 60°C; 60 sec 72°C with final incubation of 20 minutes at 72°C.

0.75 µl of amplified product were added to 13.5 µl of deionized formamide, 0.75 µl of water and 0.5 µl of size standard (500Rox), and the fragments were analysed with a sequencer AbiPrism 310 using the 47 cm capillary and polymer POP-6. The following run parameters were set: injection time 30 sec, injection voltage 3.0 kV, run temperature 60°C and run time 30 min.

The peaks resulting from the analysis of the fragments, before their normalization, are shown in Figure 11 and indicate the result obtained.

For each sample the peaks corresponding to the 27 pairs of ligated and amplified probes were selected (24 peaks representing probes for the gene FECH and 3 for inner controls) and the values of their heights were exported into an Excel file. After ordering the height values of the peaks corresponding to the gene involved according to their position on the chromosome, said value was divided by the sum of the height values of the control peaks (normalization).

For each sample the values of the normalized peaks were divided by the median obtained from the height values of the corresponding normalized values in normal subjects (calibration). A graph was created for each sample for displaying the results. Peaks from 0.8 to 1.2 are present in double copy in the genome, whereas if they are around 0.4-0.5 they are in single copy, around 1.5-1.6 they are in triple copy. Some examples are shown in Figure III; wherein the graphs CRTL1-3 represent results obtained from normal subjects and required for calibration.

The results show that 11 out of 15 analysed patients are normal for the gene FECH, whereas 4 of them show abnormalities in the gene FECH and surrounding regions. As can be seen in Figure 3, patients 399, 844 and 909 have 3 different deletions in the gene FECH.

Said result was confirmed with other molecular techniques, such as long PCR and deletion breakpoint sequencing.

### Example 2 - Diagnosis of the presence of Porphyria-related abnormalities in gene HMBS

The same protocol as in Example 1 was followed using 2.4 µl of each probe (probes L: SEQ ID NO. 283+ SEQ ID NOs. 25-47 and probes R: SEQ ID NOs. 166-188+ SEQ ID NO. 284) with a concentration of 1 pmol/µl (HMBS Probe Mix). HMBS Probe was also added with the 3 pairs of inner control probes (probes L: SEQ ID NO. 283+ SEQ ID NOs. 285, 287, 289 and probes R: SEQ ID NOs. 286, 288, 290+ SEQ ID NO. 284) in a total final volume of 600 µl of TE. An analysis was carried out on samples from 30 subjects, 16 healthy and 14 with clinical symptoms which can be related to Porphyria. Three patients (patients 496, 526 and 642) showed an abnormality in the gene HMBS. Figure 4 discloses some examples of the results.

## Claims

1. An association of probes comprising a group of probes having at one end of the sequence a tag and at the other end a sequence hybridising to at least one part of the sequence of the FECH gene said hybridising sequences being SEQ ID NO: 1-24 and a group of probes having at one end of the sequence a tag and at the other end a sequence hybridising to at least one part of the sequence of the FECH gene said hybridising sequences being SEQ ID NO: 142- 165.

2. The association of probes according to claim 1, wherein the tag is SEQ ID NO. 283 or SEQ ID NO: 284.

3. The association of probes according to claim 1 or 2, wherein the tag is present at 5' end and the hybridising sequence is present at 3' end.

4. The association of probes according to any one of claims 1-3, wherein the tag is present at 5' end of SEQ ID NO: 1-24.

5. The association according to any one of claims 1-4, wherein the tag at 5' end of SEQ ID NO: 1-24 is SEQ ID NO: 283.

6. The association of probes according to any one of claims 1-5, wherein the tag is present at 3' end and the hybridising sequence is present at 5' end.

7. The association of probes according to any one of claims 1-6, wherein the tag is present at 3' end of SEQ ID NO: 142-165.

8. The association according to any one of claims 1-6, wherein the tag at 3' end of SEQ 1D NO: 142- 165 is SEQ ID NO: 284.

9. A method for determining the presence of Erythropoietic Protoporphyria in a sample of biological tissue including the following steps:
(i) extracting nucleic acid from a sample;
(ii) introducing the association of probes according to any one of claims 1-8 into the nucleic acid extracted according to step (i), and reacting in the presence of at least one enzyme ligase in at least one ligation reaction;
(iii) performing at least one amplification reaction on the probes obtained from (ii); and
(iv) determining the presence and amount of nucleotide sequences resulting from ligation and amplification.

10. The method according to claim 8, wherein a pair of probes for inner control is introduced into step (ii), said pair of probes having preferably hybridising sequences: SEQ ID NO: 285-286 and/or 287-288 and/or 289-290, and the same tag of the association of probes present in step (ii).

11. The method according to claim 10, wherein the information given by the pair of probes having hybridising sequences SEQ ID NO: 285-286 and/or 287-288 and/or 289-290 is used to normalize the data determined according to step (iv).

12. Use of the association of probes according to any one of claims 1-8 in a ligation reaction and a PCR reaction for diagnosing the presence of porphyric abnormalities.

13. The use according to claim 12 for diagnosing abnormalities of gene FECH.

14. A kit for diagnosing Erythropoietic Protoporphyria including at least one compartment containing the reagents for performing at least one ligation and at least one PCR reaction, and at least one compartment containing the association of probes according to any one of claims 1-8.

## Patentansprüche

1. Verband von Sonden, umfassend eine Gruppe von Sonden, aufweisend an einem Ende der Sequenz eine Markierung und am anderen Ende eine Sequenz, hybridisierend zu mindestens einem Teil der Sequenz des FECH-Gens, wobei es sich bei diesen hybridisierenden Sequenzen um SEQ ID NO: 1-24 handelt, und eine Gruppe von Sonden, aufweisend an einem Ende der Sequenz eine Markierung und am anderen Ende eine Sequenz, hybridisierend zu mindestens einem Teil der Sequenz des FECH-Gens, wobei es sich bei den hybridisierenden Sequenzen um SEQ ID NO: 142-165 handelt.

2. Verband von Sonden nach Anspruch 1, wobei die Markierung SEQ ID NO: 283 oder SEQ ID NO: 284 ist.

3. Verband von Sonden nach Anspruch 1 oder 2, wobei die Markierung am Ende 5' und die hybridisierende Sequenz am Ende 3' vorhanden ist.

4. Verband von Sonden nach einem der Ansprüche 1 bis 3, wobei die Markierung am Ende 5' der SEQ ID NO: 1-24 vorhanden ist.

5. Verband von Sonden nach einem der Ansprüche 1 bis 4, wobei die Markierung am Ende 5' der SEQ ID NO: 1-24 die SEQ ID NO: 283 ist.

6. Verband von Sonden nach einem der Ansprüche 1 bis 5, wobei die Markierung am Ende 3' vorhanden ist und die hybridisierende Sequenz am Ende 5' vorhanden ist.

7. Verband von Sonden nach einem der Ansprüche 1 bis 6, wobei die Markierung am Ende 3' der SEQ ID NO: 142-165 vorhanden ist.

8. Verband von Sonden nach einem der Ansprüche 1 bis 6, wobei die Markierung am Ende 3' der SEQ ID NO: 142-165 die SEQ ID NO: 284 ist.

9. Verfahren zur Ermittlung des Vorhandenseins von erythropoetischer Protoporphyrie in einer Probe biologischen Gewebes, umfassend folgende Schritte:
(i) Extraktion von Nukleinsäure aus einer Probe;
(ii) Einführen des Verbands von Sonden nach einem der Ansprüche 1 bis 8 in die Nukleinsäure, die nach Schritt (i) extrahiert wurde, und Reaktion in Gegenwart von mindestens einer Enzym-Ligase in mindestens einer Ligationsreaktion;
(iii) Durchführen von mindestens einer Amplifikationsreaktion auf den Sonden erhalten aus (II);
und
(iv) Ermitteln des Vorhandenseins und der Menge an Nukleotidsequenzen, die sich aus der Ligation und Amplifikation ergeben.

10. Verfahren nach Anspruch 8, wobei ein Paar Sonden für die interne Kontrolle in Schritt (ii) eingeführt werden, wobei dieses Paar Sonden vorzugsweise hybridisierende Sequenzen aufweist: SEQ ID NO: 285-286 und/oder 287-288 und/oder 289-290 sowie dieselbe Markierung des Verbands von Sonden aus Schritt (ii).

11. Verfahren nach Anspruch 10, wobei die Informationen, die das Paar Sonden, aufweisend hybridisierende Sequenzen SEQ ID NO: 285-286 und/oder 287-288 und/oder 289-290, liefern, verwendet werden, um die gemäß Schritt (iv) ermittelten Daten zu normalisieren.

12. Verwendung des Verbands von Sonden nach einem der Ansprüche 1 bis 8 in einer Ligationsreaktion und einer PCR-Reaktion zur Diagnose des Vorhandenseins porphyrischer Abnormalitäten.

13. Verwendung nach Anspruch 12 zur Diagnose von Abnormalitäten des FECH-Gens.

14. Set zur Diagnose erythropoetischer Protoporphyrie, umfassend mindestens einen Abschnitt, enthaltend die Reagenzien für die Durchführung von mindestens einer Ligations- und mindestens einer PCR-Reaktion, und mindestens einen Abschnitt, enthaltend den Verband von Sonden nach einem der Ansprüche 1 bis 8.

## Revendications

1. Association de sondes comprenant un groupe de sondes ayant à une extrémité de la séquence un marqueur et à l'autre extrémité une séquence qui s'hybride à au moins une partie de la séquence du gène FECH lesdites séquences qui s'hybrident étant celles indiquées dans SEQ ID N°: 1- 24 et un groupe de sondes ayant à une extrémité de la séquence un marqueur et à l'autre extrémité une séquence qui s'hybride à au moins une partie de la séquence du gène FECH lesdites séquences qui s'hybrident étant celles indiquées dans SEQ ID n°: 142 - 165.

2. Association de sondes selon la revendication 1, dans laquelle le marqueur est la séquence indiquée dans SEQ ID N° : 283 ou SEQ ID N° : 284.

3. Association de sondes selon les revendications 1 ou 2, dans laquelle le marqueur est présent à l'extrémité 5' et la séquence qui s'hybride est présente à l'extrémité 3'.

4. Association de sondes selon l'une quelconque des revendications de 1 à 3, dans laquelle le marqueur est présent à l'extrémité 5' des séquences indiquées dans SEQ ID N° : 1-24.

5. Association selon l'une quelconque des revendications de 1 à 4, dans laquelle le marqueur présent à l'extrémité 5' des séquences indiquées dans SEQ ID N° : 1-24 est la séquence indiquée dans SEQ ID N° : 283.

6. Association de sondes selon l'une quelconque des revendications de 1 à 5, dans laquelle le marqueur est présent à l'extrémité 3' et la séquence qui s'hybride est présente à l'extrémité 5'.

7. Association de sondes selon l'une quelconque des revendications de 1 à 6, dans laquelle le marqueur est présent à l'extrémité 3' des séquences indiquées dans SEQ ID N° : 142- 165.

8. Association selon l'une quelconque des revendications de 1 à 6, dans laquelle le marqueur est présent à l'extrémité 3' des séquences indiquées dans SEQ ID N° : 142 - 165 est la séquence indiquée dans SEQ ID N° : 284.

9. Procédé servant à déterminer la présence de Protoporphyrie érythropoïétique dans un échantillon de tissu biologique incluant les étapes suivantes :
(i) extraction de l'acide nucléique d'un échantillon ;
(ii) introduction de l'association de sondes selon l'une quelconque des revendications de 1 à 8 dans l'acide nucléique extrait selon l'étape (i), et réaction en présence d'au moins une enzyme ligase dans au moins une réaction de ligation ;
(iii) réalisation d'au moins une réaction d'amplification sur les sondes obtenues à partir de (II) ;
et
(iv) détermination de la présence et du nombre de séquences nucléotides résultant de la ligation et de l'amplification.

10. Procédé selon la revendication 8, dans lequel une paire de sondes, pour un contrôle interne, est introduite à l'étape (ii), ladite paire de sondes ayant de préférence les séquences qui s'hybrident suivantes indiquées dans : SEQ ID N° : 285-286 et/ou 287-288 et/ou 289-290, et le même marqueur de l'association de sondes présentes à l'étape (ii).

11. Procédé selon la revendication 10, dans lequel les informations données par la paire de sondes ayant les séquences qui s'hybrident indiquées dans SEQ ID N° : 285-286 et/ou 287-288 et/ou 289-290 sont utilisées pour normaliser les données déterminées selon l'étape (iv).

12. Utilisation de l'association de sondes selon l'une quelconque des revendications de 1 à 8 dans une réaction de ligation et une réaction ACP pour diagnostiquer la présence d'anomalies porphyriques.

13. Utilisation selon la revendication 12 permettant de diagnostiquer les anomalies du gène FECH.

14. Kit servant à diagnostiquer la Protoporphyrie érythropoïétique incluant au moins un compartiment contenant les réactifs servant à effectuer au moins une ligation et au moins une réaction ACP, et au moins un compartiment contenant l'association de sondes selon l'une quelconque des revendications de 1 à 8.


### SEQUENCE LISTING

<110>FONDAZIONE IRCCS "OSPEDALE MAGGIORE POLICLINICO
   MANGIAGALLI E REGINA ELENA" UNIVERSITÀ DEGLI STUDI DI MILANO
<120>Probes for use in diagnosing porphyria and allelic quantification of porphyria related genes by ligation and amplification reactions
<130> 21.O0107.12.WO.10
<160> 291
<170> PatentIn version 3.5
<210> 1
   <211> 32
   <212> DNA
   <213> Artificial
<220>
   <223> Detection Probe
<400> 1
   ctgaagacac acggttagta aaaggcagaa cc 32
<210> 2
   <211> 46
   <212> DNA
   <213> Artificial
<220>
   <223> Detection Probe
<400> 2
   caaatgaaga gtgaatcccg ggaaaagaaa gaggtgagtc ctgacc 46
<210> 3
   <211> 25
   <212> DNA
   <213> Artificial
<220>
   <223> Detection Probe
<400> 3
   cttttaaaaa atgcaggctc caggt 25
<210> 4
   <211> 44
   <212> DNA
   <213> Artificial
<220>
   <223> Detection Probe
<400> 4
   cttggaacga ttcttaacgt ggattctgaa taggtatcac atcc 44
<210> 5
   <211> 25
   <212> DNA
   <213> Artificial
<220>
   <223> Detection Probe
<400> 5
   ccagtcctga gccccctagt gtaat 25
<210> 6
   <211> 28
   <212> DNA
   <213> Artificial
<220>
   <223> Detection Probe
<400> 6
   cctccaagaa atgcacttgc caggctgg 28
<210> 7
   <211> 43
   <212> DNA
   <213> Artificial
<220>
   <223> Detection Probe
<400> 7
   cggtgcatat tttataagta cttttgaagt aatcctcatc tcc 43
<210> 8
   <211> 26
   <212> DNA
   <213> Artificial
<220>
   <223> Detection Probe
<400> 8
   ccatggaggt ggaagtcagg tgcagc 26
<210> 9
   <211> 38
   <212> DNA
   <213> Artificial
<220>
   <223> Detection Probe
<400> 9
   cttgactaaa gctttcactt cacattctgg aatagagg 38
<210> 10
   <211> 36
   <212> DNA
   <213> Artificial
<220>
   <223> Detection Probe
<400> 10
   caccctggaa gaaaataaga agtgtgacaa atcaac 36
<210> 11
   <211> 23
   <212> DNA
   <213> Artificial
<220>
   <223> Detection Probe
<400> 11
   ctggcaccat tcatcgccaa acg 23
<210> 12
   <211> 41
   <212> DNA
   <213> Artificial
<220>
   <223> Detection Probe
<400> 12
   cattgaacag ctgctctgag ggtacgtaag ctaggccagt g 41
<210> 13
   <211> 27
   <212> DNA
   <213> Artificial
<220>
   <223> Detection Probe
<400> 13
   ctagaaaggg ctattgcttt cacacag 27
<210> 14
   <211> 44
   <212> DNA
   <213> Artificial
<220>
   <223> Detection Probe
<400> 14
   cctttacgag tctgtttttt attttcacca taatcaaaat gcat 44
<210> 15
   <211> 30
   <212> DNA
   <213> Artificial
<220>
   <223> Detection Probe
<400> 15
   ctttatcctc ctttcttgtt actcactcag 30
<210> 16
   <211> 35
   <212> DNA
   <213> Artificial
<220>
   <223> Detection Probe
<400> 16
   ctcaggaggt aagcgccact gtccaaaaag tcatg 35
<210> 17
   <211> 38
   <212> DNA
   <213> Artificial
<220>
   <223> Detection Probe
<400> 17
   ctcaaacaga cgaatctatc aaagggcttt gtgagagg 38
<210> 18
   <211> 35
   <212> DNA
   <213> Artificial
<220>
   <223> Detection Probe
<400> 18
   cattgttctc taaggtatct acagtgttac aatcg 35
<210> 19
   <211> 27
   <212> DNA
   <213> Artificial
<220>
   <223> Detection Probe
<400> 19
   ctgtgttcca agcagctgac cctgagc 27
<210> 20
   <211> 47
   <212> DNA
   <213> Artificial
<220>
   <223> Detection Probe
<400> 20
   ctttggataa tgataaaggt ctccttgtgg ccgggtcgaa tttcagg 47
<210> 21
   <211> 52
   <212> DNA
   <213> Artificial
<220>
   <223> Detection Probe
<400> 21
   caaatgttta atcagatctg gggtggtata ctcttgccta acgcaatctg ag 52
<210> 22
   <211> 42
   <212> DNA
   <213> Artificial
<220>
   <223> Detection Probe
<400> 22
   cactattgtc tccatcgcaa cgggagatat agagacaggt aa 42
<210> 23
   <211> 49
   <212> DNA
   <213> Artificial
<220>
   <223> Detection Probe
<400> 23
   gacataaaat ggcataacaa aatatacctt gggtttcata agacaagtc 49
<210> 24
   <211> 24
   <212> DNA
   <213> Artificial
<220>
   <223> Detection Probe
<400> 24
   cgttggaggt cagtgaacac cagg 24
<210> 25
   <211> 42
   <212> DNA
   <213> Artificial
<220>
   <223> Detection Probe
<400> 25
   gccacacctg cttctggatc cgctgcttcc aagttccttt gc 42
<210> 26
   <211> 40
   <212> DNA
   <213> Artificial
<220>
   <223> Detection Probe
<400> 26
   ctgtgcaaca agttcatagc ctatagcacc gtctttgagg 40
<210> 27
   <211> 33
   <212> DNA
   <213> Artificial
<220>
   <223> Detection Probe
<400> 27
   ctggctgaca tgcccagggc tccactctca tct 33
<210> 28
   <211> 26
   <212> DNA
   <213> Artificial
<220>
   <223> Detection Probe
<400> 28
   cacccacaca cagcctactt tccaag 26
<210> 29
   <211> 36
   <212> DNA
   <213> Artificial
<220>
   <223> Detection Probe
<400> 29
   ctgaggaggg cagaaggtac tgaggaaggt taaagg 36
<210> 30
   <211> 26
   <212> DNA
   <213> Artificial
<220>
   <223> Detection Probe
<400> 30
   caggaagaaa acagcccaaa gatgag 26
<210> 31
   <211> 39
   <212> DNA
   <213> Artificial
<220>
   <223> Detection Probe
<400> 31
   cagcttgctc gcatacagac ggacagtgtg gtggcaaca 39
<210> 32
   <211> 31
   <212> DNA
   <213> Artificial
<220>
   <223> Detection Probe
<400> 32
   ctctctcctc agttgctatg tccaccacag g 31
<210> 33
   <211> 34
   <212> DNA
   <213> Artificial
<220>
   <223> Detection Probe
<400> 33
   ctgtccggca gattggagag aaaagcctgt ttac 34
<210> 34
   <211> 41
   <212> DNA
   <213> Artificial
<220>
   <223> Detection Probe
<400> 34
   ccatctctat agagtggacc tggttgttca ctccttgaag g 41
<210> 35
   <211> 38
   <212> DNA
   <213> Artificial
<220>
   <223> Detection Probe
<400> 35
   gggaaaaccc tcatgatgct gttgtctttc acccaaaa 38
<210> 36
   <211> 27
   <212> DNA
   <213> Artificial
<220>
   <223> Detection Probe
<400> 36
   gtaacttctc tctgggcagt gtggtgg 27
<210> 37
   <211> 23
   <212> DNA
   <213> Artificial
<220>
   <223> Detection Probe
<400> 37
   ccgacactgt ggtccttagc aac 23
<210> 38
   <211> 23
   <212> DNA
   <213> Artificial
<220>
   <223> Detection Probe
<400> 38
   cagatcctgc accctgagga atg 23
<210> 39
   <211> 38
   <212> DNA
   <213> Artificial
<220>
   <223> Detection Probe
<400> 39
   catcttggat ctggtgggtg tgctgcacga tcccgaga 38
<210> 40
   <211> 28
   <212> DNA
   <213> Artificial
<220>
   <223> Detection Probe
<400> 40
   ggctgcagtg tgccagtagc cgtgcata 28
<210> 41
   <211> 39
   <212> DNA
   <213> Artificial
<220>
   <223> Detection Probe
<400> 41
   ctggaggagt ctggagtcta gacggctcag atagcatac 39
<210> 42
   <211> 38
   <212> DNA
   <213> Artificial
<220>
   <223> Detection Probe
<400> 42
   catgaagatg gccctgagga tgacccacag ttggtagg 38
<210> 43
   <211> 30
   <212> DNA
   <213> Artificial
<220>
   <223> Detection Probe
<400> 43
   caatgatcaa gggactcaga catcatagaa 30
<210> 44
   <211> 52
   <212> DNA
   <213> Artificial
<220>
   <223> Detection Probe
<400> 44
   ctctaccagg ttgaagacaa tgatggaagc agaaatgact agtgactggc ca 52
<210> 45
   <211> 43
   <212> DNA
   <213> Artificial
<220>
   <223> Detection Probe
<400> 45
   cctctctaag gcaacctatg ttctgccccg ctgcacccgc cta 43
<210> 46
   <211> 53
   <212> DNA
   <213> Artificial
<220>
   <223> Detection Probe
<400> 46
   ctcttcgcct tcaagtcttt ccgggagaac tggcagcggg cttgggtgcg agc 53
<210> 47
   <211> 49
   <212> DNA
   <213> Artificial
<220>
   <223> Detection Probe
<400> 47
   cttttctatt tatatgtgtg gcttaggacc ctccgtgaac agatgatag 49
<210> 48
   <211> 50
   <212> DNA
   <213> Artificial
<220>
   <223> Detection Probe
<400> 48
   cctattggta tggcaccctc cgcccaaaac ctttcactct catccacact 50
<210> 49
   <211> 50
   <212> DNA
   <213> Artificial
<220>
   <223> Detection Probe
<400> 49
   cctttgcctg aagctgctgt gtcagtgtct caatgatctt ctgtatctag 50
<210> 50
   <211> 39
   <212> DNA
   <213> Artificial
<220>
   <223> Detection Probe
<400> 50
   ccacaggctc ccccttctcg ctagtattcc agacccagg 39
<210> 51
   <211> 48
   <212> DNA
   <213> Artificial
<220>
   <223> Detection Probe
<400> 51
   ctgaggcatt tgacttcatt gcacgggacc ctgcagagac gctacacc 48
<210> 52
   <211> 46
   <212> DNA
   <213> Artificial
<220>
   <223> Detection Probe
<400> 52
   caggtacgca atgctttcca gaatccctgc tcagcagctg caatcc 46
<210> 53
   <211> 44
   <212> DNA
   <213> Artificial
<220>
   <223> Detection Probe
<400> 53
   ctctgggtca acacatcata gccacatgtc cctactaaat attt 44
<210> 54
   <211> 25
   <212> DNA
   <213> Artificial
<220>
   <223> Detection Probe
<400> 54
   cgtgttgtgt ctgagatccc ggtgc 25
<210> 55
   <211> 23
   <212> DNA
   <213> Artificial
<220>
   <223> Detection Probe
<400> 55
   cacctgttaa tatccagccc cga 23
<210> 56
   <211> 39
   <212> DNA
   <213> Artificial
<220>
   <223> Detection Probe
<400> 56
   ctaacatcaa ggagctgttt atggagccgc ctccccgtg 39
<210> 57
   <211> 24
   <212> DNA
   <213> Artificial
<220>
   <223> Detection Probe
<400> 57
   ctaaggtgag tgctccactt gtgc 24
<210> 58
   <211> 31
   <212> DNA
   <213> Artificial
<220>
   <223> Detection Probe
<400> 58
   ctagtggaga gcagtgagcg tctgggaggc t 31
<210> 59
   <211> 30
   <212> DNA
   <213> Artificial
<220>
   <223> Detection Probe
<400> 59
   ctgcatgccc tacccactgg cctcaggtaa 30
<210> 60
   <211> 23
   <212> DNA
   <213> Artificial
<220>
   <223> Detection Probe
<400> 60
   caaagagcct gatgagactg tgc 23
<210> 61
   <211> 25
   <212> DNA
   <213> Artificial
<220>
   <223> Detection Probe
<400> 61
   cgctccttag tcctagtctc accct 25
<210> 62
   <211> 23
   <212> DNA
   <213> Artificial
<220>
   <223> Detection Probe
<400> 62
   ctttgcttcc tctgcagggc gga 23
<210> 63
   <211> 30
   <212> DNA
   <213> Artificial
<220>
   <223> Detection Probe
<400> 63
   ctctcaaatg ttttcatgct ctcaggtatc 30
<210> 64
   <211> 34
   <212> DNA
   <213> Artificial
<220>
   <223> Detection Probe
<400> 64
   ctgagtgcca tcactgcagt gtctgtagct gtgg 34
<210> 65
   <211> 23
   <212> DNA
   <213> Artificial
<220>
   <223> Detection Probe
<400> 65
   ctccagggat ttggacattt ggt 23
<210> 66
   <211> 36
   <212> DNA
   <213> Artificial
<220>
   <223> Detection Probe
<400> 66
   cagctgctac acaattagga ctgaaggaga tgccga 36
<210> 67
   <211> 28
   <212> DNA
   <213> Artificial
<220>
   <223> Detection Probe
<400> 67
   cctctcctct cttctcagaa ctgcattc 28
<210> 68
   <211> 37
   <212> DNA
   <213> Artificial
<220>
   <223> Detection Probe
<400> 68
   ctgttaatga ctgtatagag agtgggcgcc aggcagc 37
<210> 69
   <211> 42
   <212> DNA
   <213> Artificial
<220>
   <223> Detection Probe
<400> 69
   ctgaggttac tgtagacatc acgagggtga gaatagtcaa at 42
<210> 70
   <211> 50
   <212> DNA
   <213> Artificial
<220>
   <223> Detection Probe
<400> 70
   ccacattgtt gtatccgtac ctgtgtggaa ggagtagatg gggagctcag 50
<210> 71
   <211> 31
   <212> DNA
   <213> Artificial
<220>
   <223> Detection Probe
<400> 71
   ctgaggggac tgagtgagct gcctgagaaa a 31
<210> 72
   <211> 34
   <212> DNA
   <213> Artificial
<220>
   <223> Detection Probe
<400> 72
   gaataccttt aaagtttcac cacatgtgat gtag 34
<210> 73
   <211> 43
   <212> DNA
   <213> Artificial
<220>
   <223> Detection Probe
<400> 73
   cagcactgat ctaagatatt caagaccttt aggtgaatac aag 43
<210> 74
   <211> 44
   <212> DNA
   <213> Artificial
<220>
   <223> Detection Probe
<400> 74
   gtagcgtatc tcagctaaaa cccttattac ttcattgaat taac 44
<210> 75
   <211> 40
   <212> DNA
   <213> Artificial
<220>
   <223> Detection Probe
<400> 75
   gaaaagcgcc tttgtgtcta acttgtaaag acctttaatg 40
<210> 76
   <211> 23
   <212> DNA
   <213> Artificial
<220>
   <223> Detection Probe
<400> 76
   ccaacttttc tgtggaccgg tgg 23
<210> 77
   <211> 44
   <212> DNA
   <213> Artificial
<220>
   <223> Detection Probe
<400> 77
   gtgtgttttc gaaaaggctg gggtgagcat ttctgttgtt catg 44
<210> 78
   <211> 36
   <212> DNA
   <213> Artificial
<220>
   <223> Detection Probe
<400> 78
   cattttgtgc tatgggcgtg agctctgtta tccacc 36
<210> 79
   <211> 31
   <212> DNA
   <213> Artificial
<220>
   <223> Detection Probe
<400> 79
   caagcagtgg tggtttggtg gtggatgtga c 31
<210> 80
   <211> 41
   <212> DNA
   <213> Artificial
<220>
   <223> Detection Probe
<400> 80
   gtacagagct gtgccagggc tgtagttcct tcttacattc c 41
<210> 81
   <211> 30
   <212> DNA
   <213> Artificial
<220>
   <223> Detection Probe
<400> 81
   cacaaagttt ggcctcttca ctccaggatc 30
<210> 82
   <211> 29
   <212> DNA
   <213> Artificial
<220>
   <223> Detection Probe
<400> 82
   catccaaggg actgggtgcg ttgatgcag 29
<210> 83
   <211> 31
   <212> DNA
   <213> Artificial
<220>
   <223> Detection Probe
<400> 83
   ccactgtgtt tcatgtctca ctttatgatt g 31
<210> 84
   <211> 30
   <212> DNA
   <213> Artificial
<220>
   <223> Detection Probe
<400> 84
   caataccttg tctcacagtt taggtgcctc 30
<210> 85
   <211> 31
   <212> DNA
   <213> Artificial
<220>
   <223> Detection Probe
<400> 85
   ccaaatccta cctgttttag accaatccag g 31
<210> 86
   <211> 23
   <212> DNA
   <213> Artificial
<220>
   <223> Detection Probe
<400> 86
   ctacacttcc acagctccac cag 23
<210> 87
   <211> 27
   <212> DNA
   <213> Artificial
<220>
   <223> Detection Probe
<400> 87
   ctcttcctcc tcctggcaaa atcttct 27
<210> 88
   <211> 44
   <212> DNA
   <213> Artificial
<220>
   <223> Detection Probe
<400> 88
   gagggaggaa cggtaaactg gggcaggggt gtgcatagct ctag 44
<210> 89
   <211> 37
   <212> DNA
   <213> Artificial
<220>
   <223> Detection Probe
<400> 89
   cgagtaagtc cctcactctc atgccatacc tgtagtc 37
<210> 90
   <211> 37
   <212> DNA
   <213> Artificial
<220>
   <223> Detection Probe
<400> 90
   ctgtcctgcc cagagcctac ttacttgaca atggtgc 37
<210> 91
   <211> 44
   <212> DNA
   <213> Artificial
<220>
   <223> Detection Probe

<400> 91
   cagattcagg ttaaattgtg gattgagctc gcagttacag acag 44
<210> 92
   <211> 36
   <212> DNA
   <213> Artificial
<220>
   <223> Detection Probe
<400> 92
   gaaacagact acactcccgt ttggtgcatg cgccag 36
<210> 93
   <211> 28
   <212> DNA
   <213> Artificial
<220>
   <223> Detection Probe
<400> 93
   caggactttt tcagcacgtg tcgctctc 28
<210> 94
   <211> 25
   <212> DNA
   <213> Artificial
<220>
   <223> Detection Probe
<400> 94
   ctgccaccta gcaacctgtc tcctg 25
<210> 95
   <211> 25
   <212> DNA
   <213> Artificial
<220>
   <223> Detection Probe
<400> 95
   ggatccagaa gtggtagcct ctgag 25
<210> 96
   <211> 30
   <212> DNA
   <213> Artificial
<220>
   <223> Detection Probe
<400> 96
   ccatctttct atccttctct agtggaccct 30
<210> 97
   <211> 29
   <212> DNA
   <213> Artificial
<220>
   <223> Detection Probe
<400> 97
   gcattgcagc tgtttgagtc ccatgcagg 29
<210> 98
   <211> 23
   <212> DNA
   <213> Artificial
<220>
   <223> Detection Probe
<400> 98
   cggtaagcca tggaagggtg agg 23
<210> 99
   <211> 27
   <212> DNA
   <213> Artificial
<220>
   <223> Detection Probe
<400> 99
   gctggctttg cttccaggga gtgtgtg 27
<210> 100
   <211> 25
   <212> DNA
   <213> Artificial
<220>
   <223> Detection Probe
<400> 100
   ggaggagatc gggcagttgg tgaag 25
<210> 101
   <211> 33
   <212> DNA
   <213> Artificial
<220>
   <223> Detection Probe
<400> 101
   gaattgttcg gagtctcaaa gtgtctccta tag 33
<210> 102
   <211> 35
   <212> DNA
   <213> Artificial
<220>
   <223> Detection Probe
<400> 102
   gttgggcagg cagacgggtg atgctggagc ttaag 35
<210> 103
   <211> 45
   <212> DNA
   <213> Artificial
<220>
   <223> Detection Probe
<400> 103
   gcaaattctt atttgaggtt gctgtatcac aactgagtat acatg 45
<210> 104
   <211> 42
   <212> DNA
   <213> Artificial
<220>
   <223> Detection Probe
<400> 104
   gcacacccat aaagcccctg gaaaataaag caacccatat tc 42
<210> 105
   <211> 43
   <212> DNA
   <213> Artificial
<220>
   <223> Detection Probe
<400> 105
   gtctggtcag cctctcccac tagatttcaa gtattcagtt cag 43
<210> 106
   <211> 40
   <212> DNA
   <213> Artificial
<220>
   <223> Detection Probe
<400> 106
   cttattatga cagagcaatt ccaacagtca tgaaggttcc 40
<210> 107
   <211> 38
   <212> DNA
   <213> Artificial
<220>
   <223> Detection Probe
<400> 107
   caaattgctt ttcacatgtg ccacatggag ctgaagag 38
<210> 108
   <211> 38
   <212> DNA
   <213> Artificial
<220>
   <223> Detection Probe
<400> 108
   cttccacaaa tgtcttacta atcagaaggt aaaagtag 38
<210> 109
   <211> 38
   <212> DNA
   <213> Artificial
<220>
   <223> Detection Probe
<400> 109
   cacactccca atatggttct gttgaattaa gagatctg 38
<210> 110
   <211> 22
   <212> DNA
   <213> Artificial
<220>
   <223> Detection Probe
<400> 110
   cttacagtca cgtccagctt gc 22
<210> 111
   <211> 30
   <212> DNA
   <213> Artificial
<220>
   <223> Detection Probe
<400> 111
   cagtaaaagg gtgactgttg ttattgctga 30
<210> 112
   <211> 34
   <212> DNA
   <213> Artificial
<220>
   <223> Detection Probe
<400> 112
   ctgccaggca ataatgaagg ttcttttact gaag 34
<210> 113
   <211> 51
   <212> DNA
   <213> Artificial
<220>
   <223> Detection Probe
<400> 113
   ccaatccaga actatattct ttcatttcag gaattaggat tatatggact t 51
<210> 114
   <211> 47
   <212> DNA
   <213> Artificial
<220>
   <223> Detection Probe
<400> 114
   ctttctcatc ctgaagatta cgggggactc atttttacca gccccag 47
<210> 115
   <211> 37
   <212> DNA
   <213> Artificial
<220>
   <223> Detection Probe
<400> 115
   cagtctggga aaggtctctg aaagaaaaat ggaatgc 37
<210> 116
   <211> 26
   <212> DNA
   <213> Artificial
<220>
   <223> Detection Probe
<400> 116
   ccagtgagta aaattggcct ggatac 26
<210> 117
   <211> 25
   <212> DNA
   <213> Artificial
<220>
   <223> Detection Probe
<400> 117
   ggattaggac ccagttctct tgttc 25
<210> 118
   <211> 29
   <212> DNA
   <213> Artificial
<220>
   <223> Detection Probe
<400> 118
   ctcagcactg cctcttctat ttccctgtg 29
<210> 119
   <211> 32
   <212> DNA
   <213> Artificial
<220>
   <223> Detection Probe
<400> 119
   gtaaagtctt tcctgctttt gattgtgccc ag 32
<210> 120
   <211>34
   <212> DNA
   <213> Artificial
<220>
   <223> Detection Probe
<400> 120
   ctctggcctc acatacagtc tcaagcacat tcag 34
<210> 121
   <211> 27
   <212> DNA
   <213> Artificial
<220>
   <223> Detection Probe
<400> 121
   catcatgtga gcattcattg acacagg 27
<210> 122
   <211> 23
   <212> DNA
   <213> Artificial
<220>
   <223> Detection Probe
<400> 122
   ctctgtggaa gccagcttaa acc 23
<210> 123
   <211> 44
   <212> DNA
   <213> Artificial
<220>
   <223> Detection Probe
<400> 123
   gggacagccc tgcaaggttc cccaacaccg caatagtaag gatc 44
<210> 124
   <211> 41
   <212> DNA
   <213> Artificial
<220>
   <223> Detection Probe
<400> 124
   cagctggtac tggctttatg aaaatcgaaa caataggaca c 41
<210> 125
   <211> 51
   <212> DNA
   <213> Artificial
<220>
   <223> Detection Probe
<400> 125
   gaaaaccgga ttttcagtag ctgaagaaaa tatggcactg taagttaaaa c 51
<210> 126
   <211> 42
   <212> DNA
   <213> Artificial
<220>
   <223> Detection Probe
<400> 126
   ctcagtgcta acaactttgc aatgaaagga aagcggaaga cg 42
<210> 127
   <211> 24
   <212> DNA
   <213> Artificial
<220>
   <223> Detection Probe
<400> 127
   ctaggctggg caaaggaatg ctgt 24
<210> 128
   <211> 25
   <212> DNA
   <213> Artificial
<220>
   <223> Detection Probe
<400> 128
   ggagttaaca caccagggtt ctagt 25
<210> 129
   <211> 25
   <212> DNA
   <213> Artificial
<220>
   <223> Detection Probe
<400> 129
   tccaacctca tctaccccat ctttg 25
<210> 130
   <211> 43
   <212> DNA
   <213> Artificial
<220>
   <223> Detection Probe
<400> 130
   cttcccacag ctaccaatcc atatcccacc cccgctcttg cag 43
<210> 131
   <211> 41
   <212> DNA
   <213> Artificial
<220>
   <223> Detection Probe
<400> 131
   gcctacgctg tgtcttgatc tttggcgtcc ccagcagagt t 41
<210> 132
   <211> 39
   <212> DNA
   <213> Artificial
<220>
   <223> Detection Probe
<400> 132
   cgaggagtcc ccagctattg aggcaatcca tctgttgag 39
<210> 133
   <211> 23
   <212> DNA
   <213> Artificial
<220>
   <223> Detection Probe
<400> 133
   gctccccaaa acccagtcat ctg 23
<210> 134
   <211> 38
   <212> DNA
   <213> Artificial
<220>
   <223> Detection Probe
<400> 134
   caaggcaggt gagtgaacca ccagcaggga tgggcacc 38
<210> 135
   <211> 40
   <212> DNA
   <213> Artificial
<220>
   <223> Detection Probe
<400> 135
   gatgtcaggt ggtagccccg tcggacatga tggatggacg 40
<210> 136
   <211> 36
   <212> DNA
   <213> Artificial
<220>
   <223> Detection Probe
<400> 136
   cataggtatc ggtgatgagc tacagtgcca aatttg 36
<210> 137
   <211> 25
   <212> DNA
   <213> Artificial
<220>
   <223> Detection Probe
<400> 137
   caccctgatg actctgcttt gcagg 25
<210> 138
   <211> 28
   <212> DNA
   <213> Artificial
<220>
   <223> Detection Probe
<400> 138
   gacatgctca tggtgaagcc gggaatgc 28
<210> 139
   <211> 44
   <212> DNA
   <213> Artificial
<220>
   <223> Detection Probe
<400> 139
   catttcagcc tgtctccctg tctgcttccc tgcagcaccc tgac 44
<210> 140
   <211> 47
   <212> DNA
   <213> Artificial
<220>
   <223> Detection Probe
<400> 140
   gcaactttct gcatctctcc caacacaggt gctgacatca tcatcac 47
<210> 141
   <211> 24
   <212> DNA
   <213> Artificial
<220>
   <223> Detection Probe
<400> 141
   ctgacccaca aggcctggaa agag 24
<210> 142
   <211> 34
   <212> DNA
   <213> Artificial
<220>
   <223> Detection Probe
<220>
   <221> modified_base
   <222> (1)..(1)
   <223> Phosphorylation at 5'
<400> 142
   caggtttgta ttccgaagcc ctcgttcttt ccac 34
<210> 143
   <211> 40
   <212> DNA
   <213> Artificial
<220>
   <223> Detection Probe
<220>
   <221> modified_base
   <222> (1)..(1)
   <223> Phosphorylation at 5'
<400> 143
   ttgccatcgt gaacgttgtt aaagttagaa ctctggcaac 40
<210> 144
   <211> 21
   <212> DNA
   <213> Artificial
<220>
   <223> Detection Probe
<220>
   <221> modified_base
   <222> (1)..(1)
   <223> Phosphorylation at 5'
<400> 144
   ggccatgagc tgagttgtga c 21
<210> 145
   <211> 40
   <212> DNA
   <213> Artificial
<220>
   <223> Detection Probe
<220>
   <221> modified_base
   <222> (1)..(1)
   <223> Phosphorylation at 5'
<400> 145
   gagacgtgtg cttataccct cgatttgtcc agcgttgcac 40
<210> 146
   <211> 33
   <212> DNA
   <213> Artificial
<220>
   <223> Detection Probe
<220>
   <221> modified_base
   <222> (1)..(1)
   <223> Phosphorylation at 5'
<400> 146
   ctggatggtt tttgtttaat acggatggcc agc 33
<210> 147
   <211> 36
   <212> DNA
   <213> Artificial
<220>
   <223> Detection Probe
<220>
   <221> modified_base
   <222> (1)..(1)
   <223> Phosphorylation at 5'
<400> 147
   ccttcacttt tcctaatgct cttcagtgag tttcac 36
<210> 148
   <211> 47
   <212> DNA
   <213> Artificial
<220>
   <223> Detection Probe
<220>
   <221> modified_base
   <222> (1)..(1)
   <223> Phosphorylation at 5'
<400> 148
   tatgtgatag aaaagtgcca ttatagattt caatacttgt gtatctc 47
<210> 149
   <211> 24
   <212> DNA
   <213> Artificial
<220>
   <223> Detection Probe
<220>
   <221> modified_base
   <222> (1)..(1)
   <223> Phosphorylation at 5'
<400> 149
   tgcagcggcc gtcaccacag aaac 24
<210> 150
   <211> 40
   <212> DNA
   <213> Artificial
<220>
   <223> Detection Probe
<220>
   <221> modified_base
   <222> (1)..(1)
   <223> Phosphorylation at 5'
<400> 150
   ggtgtcagac tggggcttac taatgcgcta ttcatcatgc 40
<210> 151
   <211> 36
   <212> DNA
   <213> Artificial
<220>
   <223> Detection Probe
<220>
   <221> modified_base
   <222> (1)..(1)
   <223> Phosphorylation at 5'
<400> 151
   cgttgtattt tattttatat aggaagccga aaactg 36
<210> 152
   <211> 29
   <212> DNA
   <213> Artificial
<220>
   <223> Detection Probe
<220>
   <221> modified_base
   <222> (1)..(1)
   <223> Phosphorylation at 5'
<400> 152
   ccgaaccccc aagattcaag agcagtacc 29
<210> 153
   <211> 27
   <212> DNA
   <213> Artificial
<220>
   <223> Detection Probe
<220>
   <221> modified_base
   <222> (1)..(1)
   <223> Phosphorylation at 5'
<400> 153
   agtgatgtgt tttgggctca ctctggc 27
<210> 154
   <211> 29
   <212> DNA
   <213> Artificial
<220>
   <223> Detection Probe
<220>
   <221> modified_base
   <222> (1)..(1)
   <223> Phosphorylation at 5'
<400> 154
   tatccacagt acagctgctc caccacagg 29
<210> 155
   <211> 32
   <212> DNA
   <213> Artificial
<220>
   <223> Detection Probe
<220>
   <221> modified_base
   <222> (1)..(1)
   <223> Phosphorylation at 5'
<400> 155
   ctactaagtg gtctctgtat atttcaggca gc 32
<210> 156
   <211> 30
   <212> DNA
   <213> Artificial
<220>
   <223> Detection Probe
<220>
   <221> modified_base
   <222> (1)..(1)
   <223> Phosphorylation at 5'
<400> 156
   tgctttgcag atcatattct aaaggaactg 30
<210> 157
   <211> 27
   <212> DNA
   <213> Artificial
<220>
   <223> Detection Probe
<220>
   <221> modified_base
   <222> (1)..(1)
   <223> Phosphorylation at 5'
<400> 157
   gaaaggctgg agtactgcaa cccctac 27
<210> 158
   <211> 32
   <212> DNA
   <213> Artificial
<220>
   <223> Detection Probe
<220>
   <221> modified_base
   <222> (1)..(1)
   <223> Phosphorylation at 5'
<400> 158
   gggaggaaga atatcctctt ggttccgata gc 32
<210> 159
   <211> 39
   <212> DNA
   <213> Artificial
<220>
   <223> Detection Probe
<220>
   <221> modified_base
   <222> (1)..(1)
   <223> Phosphorylation at 5'
<400> 159
   ttttagtaga acataccaga gagtaatcct ctgagaaac 39
<210> 160
   <211> 27
   <212> DNA
   <213> Artificial
<220>
   <223> Detection Probe
<220>
   <221> modified_base
   <222> (1)..(1)
   <223> Phosphorylation at 5'
<400> 160
   tgtccgctct gtgtcaatcc tgtctgc 27
<210> 161
   <211> 33
   <212> DNA
   <213> Artificial
<220>
   <223> Detection Probe
<220>
   <221> modified_base
   <222> (1)..(1)
   <223> Phosphorylation at 5'
<400> 161
   gccagccaaa acaaacatgc aagggaaatg cag 33
<210> 162
   <211> 42
   <212> DNA
   <213> Artificial
<220>
   <223> Detection Probe
<220>
   <221> modified_base
   <222> (1)..(1)
   <223> Phosphorylation at 5'
<400> 162
   gtttttctgg aattttggtc tgactttggt ttggacagtg cc 42
<210> 163
   <211> 50
   <212> DNA
   <213> Artificial
<220>
   <223> Detection Probe
<220>
   <221> modified_base
   <222> (1)..(1)
   <223> Phosphorylation at 5'
<400> 163
   cttttctcaa agatggggct gctcctcctg agtccaagct tggcaaggcc 50
<210> 164
   <211> 39
   <212> DNA
   <213> Artificial
<220>
   <223> Detection Probe
<220>
   <221> modified_base
   <222> (1)..(1)
   <223> Phosphorylation at 5'
<400> 164
   ttcttggcaa agtgtaacca gccagaccca tgcattgcc 39
<210> 165
   <211> 24
   <212> DNA
   <213> Artificial
<220>
   <223> Detection Probe
<220>
   <221> modified_base
   <222> (1)..(1)
   <223> Phosphorylation at 5'
<400> 165
   cgggacttct tctgggaatt gttc 24
<210> 166
   <211> 32
   <212> DNA
   <213> Artificial
<220>
   <223> Detection Probe
<220>
   <221> modified_base
   <222> (1)..(1)
   <223> Phosphorylation at 5'
<400> 166
   ctccctcctg gcatcactgt ctgcgactca gg 32
<210> 167
   <211> 48
   <212> DNA
   <213> Artificial
<220>
   <223> Detection Probe
<220>
   <221> modified_base
   <222> (1)..(1)
   <223> Phosphorylation at 5'
<400> 167
   atgtagtgga tgtgcttgct gagtggggct ccctgtacgt gctgacgc 48
<210> 168
   <211> 27
   <212> DNA
   <213> Artificial
<220>
   <223> Detection Probe
<220>
   <221> modified_base
   <222> (1)..(1)
   <223> Phosphorylation at 5'
<400> 168
   aatgtcacag ccctcagaac taaagcg 27
<210> 169
   <211> 26
   <212> DNA
   <213> Artificial
<220>
   <223> Detection Probe
<220>
   <221> modified_base
   <222> (1)..(1)
   <223> Phosphorylation at 5'
<400> 169
   cggagccatg tctggtaacg gcaatg 26
<210> 170
   <211> 42
   <212> DNA
   <213> Artificial
<220>
   <223> Detection Probe
<220>
   <221> modified_base
   <222> (1)..(1)
   <223> Phosphorylation at 5'
<400> 170
   gaccagcctt ggagtatttc cccactctga gactcagctg gc 42
<210> 171
   <211> 28
   <212> DNA
   <213> Artificial
<220>
   <223> Detection Probe
<220>
   <221> modified_base
   <222> (1)..(1)
   <223> Phosphorylation at 5'
<400> 171
   agtgattcgc gtgggtaccc gcaagagc 28
<210> 172
   <211> 25
   <212> DNA
   <213> Artificial
<220>
   <223> Detection Probe
<220>
   <221> modified_base
   <222> (1)..(1)
   <223> Phosphorylation at 5'
<400> 172
   ttgaaagcct cgtaccctgg cctgc 25
<210> 173
   <211> 31
   <212> DNA
   <213> Artificial
<220>
   <223> Detection Probe
<220>
   <221> modified_base
   <222> (1)..(1)
   <223> Phosphorylation at 5'

<400> 173
   ggacaagatt cttgatactg cactctctaa g 31
<210> 174
   <211> 32
   <212> DNA
   <213> Artificial
<220>
   <223> Detection Probe
<220>
   <221> modified_base
   <222> (1)..(1)
   <223> Phosphorylation at 5'
<400> 174
   caaggagctt gaacatgccc tggagaagaa tg 32
<210> 175
   <211> 31
   <212> DNA
   <213> Artificial
<220>
   <223> Detection Probe
<220>
   <221> modified_base
   <222> (1)..(1)
   <223> Phosphorylation at 5'
<400> 175
   acctgcccac tgtgcttcct cctggcttca c 31
<210> 176
   <211> 38
   <212> DNA
   <213> Artificial
<220>
   <223> Detection Probe
<220>
   <221> modified_base
   <222> (1)..(1)
   <223> Phosphorylation at 5'
<400> 176
   tttgttggga agaccctaga aaccctgcca gagaagag 38
<210> 177
   <211> 21
   <212> DNA
   <213> Artificial
<220>
   <223> Detection Probe
<220>
   <221> modified_base
   <222> (1)..(1)
   <223> Phosphorylation at 5'
<400> 177
   gaaccagctc cctgcgaaga g 21
<210> 178
   <211> 27
   <212> DNA
   <213> Artificial
<220>
   <223> Detection Probe
<220>
   <221> modified_base
   <222> (1)..(1)
   <223> Phosphorylation at 5'
<400> 178
   tctccacagc ggggaaacct caacacc 27
<210> 179
   <211> 23
   <212> DNA
   <213> Artificial
<220>
   <223> Detection Probe
<220>
   <221> modified_base
   <222> (1)..(1)
   <223> Phosphorylation at 5'
<400> 179
   catgtatgct gtgggccagg tac 23
<210> 180
   <211> 32
   <212> DNA
   <213> Artificial
<220>
   <223> Detection Probe
<220>
   <221> modified_base
   <222> (1)..(1)
   <223> Phosphorylation at 5'
<400> 180
   ctctgcttcg ctgcatcgct gaaagggcct tc 32
<210> 181
   <211> 28
   <212> DNA
   <213> Artificial
<220>
   <223> Detection Probe
<220>
   <221> modified_base
   <222> (1)..(1)
   <223> Phosphorylation at 5'
<400> 181
   cagctatgaa ggatgggcaa gtaagtgg 28
<210> 182
   <211> 29
   <212> DNA
   <213> Artificial
<220>
   <223> Detection Probe
<220>
   <221> modified_base
   <222> (1)..(1)
   <223> Phosphorylation at 5'
<400> 182
   aagagaccat gcaggctacc atccatgtc 29
<210> 183
   <211> 42
   <212> DNA
   <213> Artificial
<220>
   <223> Detection Probe
<220>
   <221> modified_base
   <222> (1)..(1)
   <223> Phosphorylation at 5'
<400> 183
   catcactgct cgtaacattc cacgagggcc ccagttggct gc 42
<210> 184
   <211> 28
   <212> DNA
   <213> Artificial
<220>
   <223> Detection Probe
<220>
   <221> modified_base
   <222> (1)..(1)
   <223> Phosphorylation at 5'
<400> 184
   gagtcgaacg agctgatatc gaatggag 28
<210> 185
   <211> 38
   <212> DNA
   <213> Artificial
<220>
   <223> Detection Probe
<220>
   <221> modified_base
   <222> (1)..(1)
   <223> Phosphorylation at 5'
<400> 185
   gcctctaggc cgttaattcc tgctaggata ttgatggc 38
<210> 186
   <211> 43
   <212> DNA
   <213> Artificial
<220>
   <223> Detection Probe
<220>
   <221> modified_base
   <222> (1)..(1)
   <223> Phosphorylation at 5'
<400> 186
   tctactgttt ccgcccggat cttgttcgct gaacaaccat tac 43
<210> 187
   <211> 39
   <212> DNA
   <213> Artificial
<220>
   <223> Detection Probe
<220>
   <221> modified_base
   <222> (1)..(1)
   <223> Phosphorylation at 5'
<400> 187
   gctgaacgag caggcctgca gaaacggggt gagttggac 39
<210> 188
   <211> 35
   <212> DNA
   <213> Artificial
<220>
   <223> Detection Probe
<220>
   <221> modified_base
   <222> (1)..(1)
   <223> Phosphorylation at 5'
<400> 188
   agggcatctc tcccaggtga cccttctttt ctgtc 35
<210> 189
   <211> 44
   <212> DNA
   <213> Artificial
<220>
   <223> Detection Probe
<220>
   <221> modified_base
   <222> (1)..(1)
   <223> Phosphorylation at 5'
<400> 189
   ctcgaaaaac aaaagcctaa gcagatttct caggcaattt atcc 44
<210> 190
   <211> 40
   <212> DNA
   <213> Artificial
<220>
   <223> Detection Probe
<220>
   <221> modified base
   <222> (1)..(1)
   <223> Phosphorylation at 5'
<400> 190
   aggacaagtg acagggatta tataaggaat tcaggactcc 40
<210> 191
   <211> 37
   <212> DNA
   <213> Artificial
<220>
   <223> Detection Probe
<220>
   <221> modified_base
   <222> (1)..(1)
   <223> Phosphorylation at 5'
<400> 191
   tgggtaccct gcttcgcgtt agccactcct cacctgc 37
<210> 192
   <211> 38
   <212> DNA
   <213> Artificial
<220>
   <223> Detection Probe
<220>
   <221> modified_base
   <222> (1)..(1)
   <223> Phosphorylation at 5'
<400> 192
   tgtctgaacc actgggtggg aaacttttgg aagaatac 38
<210> 193
   <211> 38
   <212> DNA
   <213> Artificial
<220>
   <223> Detection Probe
<220>
   <221> modified_base
   <222> (1)..(1)
   <223> Phosphorylation at 5'
<400> 193
   ccaccgtact gaaagggcag ggaaagaacc gatgagac 38
<210> 194
   <211> 48
   <212> DNA
   <213> Artificial
<220>
   <223> Detection Probe
<220>
   <221> modified_base
   <222> (1)..(1)
   <223> Phosphorylation at 5'
<400> 194
   ttttacaagt cagtggtagt accagtttag agtaaagcag atctgttc 48
<210> 195
   <211> 39
   <212> DNA
   <213> Artificial
<220>
   <223> Detection Probe
<220>
   <221> modified_base
   <222> (1)..(1)
   <223> Phosphorylation at 5'
<400> 195
   tgaagactaa cgccggaccc cgagatcgtg agttgtggg 39
<210> 196
   <211> 23
   <212> DNA
   <213> Artificial
<220>
   <223> Detection Probe
<220>
   <221> modified_base
   <222> (1)..(1)
   <223> Phosphorylation at 5'
<400> 196
   gtgggatcca agctaccatt tgc 23
<210> 197
   <211> 35
   <212> DNA
   <213> Artificial
<220>
   <223> Detection Probe
<220>
   <221> modified_base
   <222> (1)..(1)
   <223> Phosphorylation at 5'
<400> 197
   gactggcctt aagtgtccct atctattctc cctac 35
<210> 198
   <211> 28
   <212> DNA
   <213> Artificial
<220>
   <223> Detection Probe
<220>
   <221> modified_base
   <222> (1)..(1)
   <223> Phosphorylation at 5'
<400> 198
   cagagggagc ttcatttaat gctcttcc 28
<210> 199
   <211> 31
   <212> DNA
   <213> Artificial
<220>
   <223> Detection Probe
<220>
   <221> modified_base
   <222> (1)..(1)
   <223> Phosphorylation at 5'
<400> 199
   ggattcgctc cgttcgaggc cctaatggtg c 31
<210> 200
   <211> 30
   <212> DNA
   <213> Artificial
<220>
   <223> Detection Probe
<220>
   <221> modified_base
   <222> (1)..(1)
   <223> Phosphorylation at 5'
<400> 200
   caccagcacc tccgctcctt ttactgtgcc 30
<210> 201
   <211> 43
   <212> DNA
   <213> Artificial
<220>
   <223> Detection Probe
<220>
   <221> modified_base
   <222> (1)..(1)
   <223> Phosphorylation at 5'
<400> 201
   acagttttgc ccagcgccgc cttggacctg aggtgacact tgc 43
<210> 202
   <211> 31
   <212> DNA
   <213> Artificial
<220>
   <223> Detection Probe
<220>
   <221> modified_base
   <222> (1)..(1)
   <223> Phosphorylation at 5'
<400> 202
   taaggtggcg tctctagcca tggacagtct c 31
<210> 203
   <211> 27
   <212> DNA
   <213> Artificial
<220>
   <223> Detection Probe
<220>
   <221> modified_base
   <222> (1)..(1)
   <223> Phosphorylation at 5'
<400> 203
   ccccacagcc agactcagca ctcattc 27
<210> 204
   <211> 24
   <212> DNA
   <213> Artificial
<220>
   <223> Detection Probe
<220>
   <221> modified_base
   <222> (1)..(1)
   <223> Phosphorylation at 5'
<400> 204
   tctaagggac agcagtctgg aggc 24
<210> 205
   <211> 36
   <212> DNA
   <213> Artificial
<220>
   <223> Detection Probe
<220>
   <221> modified_base
   <222> (1)..(1)
   <223> Phosphorylation at 5'
<400> 205
   tgaatctgca gtaccaagga gcccatctgc ctgtcc 36
<210> 206
   <211> 25
   <212> DNA
   <213> Artificial
<220>
   <223> Detection Probe
<220>
   <221> modified_base
   <222> (1)..(1)
   <223> Phosphorylation at 5'
<400> 206
   gccatcttca gaagatccag gagtc 25
<210> 207
   <211> 42
   <212> DNA
   <213> Artificial
<220>
   <223> Detection Probe
<220>
   <221> modified_base
   <222> (1)..(1)
   <223> Phosphorylation at 5'
<400> 207
   gccactgctt ggtccatcta cacaaggtaa gttgggataa ac 42
<210> 208
   <211> 30
   <212> DNA
   <213> Artificial
<220>
   <223> Detection Probe
<220>
   <221> modified_base
   <222> (1)..(1)
   <223> Phosphorylation at 5'
<400> 208
   cccagtatac actaggtcac tggcaaaaac 30
<210> 209
   <211> 31
   <212> DNA
   <213> Artificial
<220>
   <223> Detection Probe
<220>
   <221> modified_base
   <222> (1)..(1)
   <223> Phosphorylation at 5'
<400> 209
   agtcagtgtc ctgggcacag aacctaacag c 31
<210> 210
   <211> 46
   <212> DNA
   <213> Artificial
<220>
   <223> Detection Probe
<220>
   <221> modified base
   <222> (1)..(1)
   <223> Phosphorylation at 5'
<400> 210
   gtcggtccct gatctcggcc aaatagggca ctgagacttc ggaagc 46
<210> 211
   <211> 30
   <212> DNA
   <213> Artificial
<220>
   <223> Detection Probe
<220>
   <221> modified_base
   <222> (1)..(1)
   <223> Phosphorylation at 5'
<400> 211
   gatcacctga atcccctcag ccttctgagc 30
<210> 212
   <211> 41
   <212> DNA
   <213> Artificial
<220>
   <223> Detection Probe
<220>
   <221> modified_base
   <222> (1)..(1)
   <223> Phosphorylation at 5'
<400> 212
   gagggtaggg aacaggtaag gaggcatcaa gagggaagaa c 41
<210> 213
   <211> 36
   <212> DNA
   <213> Artificial
<220>
   <223> Detection Probe
<220>
   <221> modified_base
   <222> (1)..(1)
   <223> Phosphorylation at 5'
<400> 213
   gttcttgata aggattcttt attaggctca agtctc 36
<210> 214
   <211> 35
   <212> DNA
   <213> Artificial
<220>
   <223> Detection Probe
<220>
   <221> modified_base
   <222> (1)..(1)
   <223> Phosphorylation at 5'
<400> 214
   cacctacttt caatgggtat gagtattagg ttgtc 35
<210> 215
   <211> 36
   <212> DNA
   <213> Artificial
<220>
   <223> Detection Probe
<220>
   <221> modified_base
   <222> (1)..(1)
   <223> Phosphorylation at 5'
<400> 215
   cttttaacca gcctcccttt ttctaagcct ttagtg 36
<210> 216
   <211> 44
   <212> DNA
   <213> Artificial
<220>
   <223> Detection Probe
<220>
   <221> modified_base
   <222> (1)..(1)
   <223> Phosphorylation at 5'
<400> 216
   gatgtttgat aatgccaaag atgatggtga ctaacagctg tttg 44
<210> 217
   <211> 23
   <212> DNA
   <213> Artificial
<220>
   <223> Detection Probe
<220>
   <221> modified_base
   <222> (1)..(1)
   <223> Phosphorylation at 5'
<400> 217
   gagaggaagg aaggtaagga ggc 23
<210> 218
   <211> 32
   <212> DNA
   <213> Artificial
<220>
   <223> Detection Probe
<220>
   <221> modified_base
   <222> (1)..(1)
   <223> Phosphorylation at 5'
<400> 218
   gaaatctttc agaggaagct gcaaaacaaa tg 32
<210> 219
   <211> 32
   <212> DNA
   <213> Artificial
<220>
   <223> Detection Probe
<220>
   <221> modified_base
   <222> (1)..(1)
   <223> Phosphorylation at 5'
<400> 219
   ccaagaatcc tcatgctcct actatccatt tc 32
<210> 220
   <211> 31
   <212> DNA
   <213> Artificial
<220>
   <223> Detection Probe
<220>
   <221> modified_base
   <222> (1)..(1)
   <223> Phosphorylation at 5'
<400> 220
   ctcactccaa catacttgaa tcaagaagac g 31
<210> 221
   <211> 31
   <212> DNA
   <213> Artificial
<220>
   <223> Detection Probe
<220>
   <221> modified_base
   <222> (1)..(1)
   <223> Phosphorylation at 5'
<400> 221
   ccttgtgaaa aagcactgtg atgactcatt c 31
<210> 222
   <211> 36
   <212> DNA
   <213> Artificial
<220>
   <223> Detection Probe
<220>
   <221> modified_base
   <222> (1)..(1)
   <223> Phosphorylation at 5'
<400> 222
   cagaattgaa agtatcttga tgtctttacc tctaac 36
<210> 223
   <211> 27
   <212> DNA
   <213> Artificial
<220>
   <223> Detection Probe
<220>
   <221> modified_base
   <222> (1)..(1)
   <223> Phosphorylation at 5'
<400> 223
   gcagaatggc tgtgcagggg tttggag 27
<210> 224
   <211> 33
   <212> DNA
   <213> Artificial
<220>
   <223> Detection Probe
<220>
   <221> modified_base
   <222> (1)..(1)
   <223> Phosphorylation at 5'
<400> 224
   ggagattctg aagttggcaa tgtctacaat tcc 33
<210> 225
   <211> 28
   <212> DNA
   <213> Artificial
<220>
   <223> Detection Probe
<220>
   <221> modified_base
   <222> (1)..(1)
   <223> Phosphorylation at 5'
<400> 225
   cagcccctaa ccattatttg cattcaag 28
<210> 226
   <211> 29
   <212> DNA
   <213> Artificial
<220>
   <223> Detection Probe
<220>
   <221> modified_base
   <222> (1)..(1)
   <223> Phosphorylation at 5'
<400> 226
   gatattatgt atggcgacct ggtggttac 29
<210> 227
   <211> 29
   <212> DNA
   <213> Artificial
<220>
   <223> Detection Probe
<220>
   <221> modified_base
   <222> (1)..(1)
   <223> Phosphorylation at 5'
<400> 227
   gcattacact ttgaaaccta aatggaggc 29
<210> 228
   <211> 23
   <212> DNA
   <213> Artificial
<220>
   <223> Detection Probe
<220>
   <221> modified_base
   <222> (1)..(1)
   <223> Phosphorylation at 5'
<400> 228
   gcatgaatga aggtggagag agc 23
<210> 229
   <211> 40
   <212> DNA
   <213> Artificial
<220>
   <223> Detection Probe
<220>
   <221> modified_base
   <222> (1)..(1)
   <223> Phosphorylation at 5'
<400> 229
   gaacaggttt aaggtctagg ctttagagtt ggggaaaggg 40
<210> 230
   <211> 31
   <212> DNA
   <213> Artificial
<220>
   <223> Detection Probe
<220>
   <221> modified_base
   <222> (1)..(1)
   <223> Phosphorylation at 5'
<400> 230
   caggggcttt tcatatttgt cagagggctt g 31
<210> 231
   <211> 35
   <212> DNA
   <213> Artificial
<220>
   <223> Detection Probe
<220>
   <221> modified_base
   <222> (1)..(1)
   <223> Phosphorylation at 5'
<400> 231
   ccttcttcat agtaagccgt acccagtgtt ggcac 35
<210> 232
   <211> 26
   <212> DNA
   <213> Artificial
<220>
   <223> Detection Probe
<220>
   <221> modified_base
   <222> (1)..(1)
   <223> Phosphorylation at 5'
<400> 232
   ctgaccatgg aagcgaatgg gttggg 26
<210> 233
   <211> 28
   <212> DNA
   <213> Artificial
<220>
   <223> Detection Probe
<220>
   <221> modified_base
   <222> (1)..(1)
   <223> Phosphorylation at 5'
<400> 233
   gcaggccgtt acttaccagg taagagtc 28
<210> 234
   <211> 26
   <212> DNA
   <213> Artificial
<220>
   <223> Detection Probe
<220>
   <221> modified_base
   <222> (1)..(1)
   <223> Phosphorylation at 5'
<400> 234
   ctgaggcctg ctgtgaactg actctg 26
<210> 235
   <211> 25
   <212> DNA
   <213> Artificial
<220>
   <223> Detection Probe
<220>
   <221> modified_base
   <222> (1)..(1)
   <223> Phosphorylation at 5'
<400> 235
   tttcctacag ccactgcgtc gcttc 25
<210> 236
   <211> 27
   <212> DNA
   <213> Artificial
<220>
   <223> Detection Probe
<220>
   <221> modified_base
   <222> (1)..(1)
   <223> Phosphorylation at 5'
<400> 236
   ctaggctatg tgttccaagc catcacc 27
<210> 237
   <211> 26
   <212> DNA
   <213> Artificial
<220>
   <223> Detection Probe
<220>
   <221> modified_base
   <222> (1)..(1)
   <223> Phosphorylation at 5'
<400> 237
   gatgacatac atggttgagg gtggtg 26
<210> 238
   <211> 33
   <212> DNA
   <213> Artificial
<220>
   <223> Detection Probe
<220>
   <221> modified_base
   <222> (1)..(1)
   <223> Phosphorylation at 5'
<400> 238
   gcatcttggc ccacagctct tcaacaagtt tgc 33
<210> 239
   <211> 23
   <212> DNA
   <213> Artificial

<220>
   <223> Detection Probe
<220>
   <221> modified_base
   <222> (1)..(1)
   <223> Phosphorylation at 5'
<400> 239
   ccttgaggtt gaggtggggg tgt 23
<210> 240
   <211> 21
   <212> DNA
   <213> Artificial
<220>
   <223> Detection Probe
<220>
   <221> modified_base
   <222> (1)..(1)
   <223> Phosphorylation at 5'
<400> 240
   gggaagacgg tgacattgca g 21
<210> 241
   <211> 33
   <212> DNA
   <213> Artificial
<220>
   <223> Detection Probe
<220>
   <221> modified_base
   <222> (1)..(1)
   <223> Phosphorylation at 5'
<400> 241
   cagatgctgg atgactttgg accacatcgc tac 33
<210> 242
   <211> 33
   <212> DNA
   <213> Artificial
<220>
   <223> Detection Probe
<220>
   <221> modified_base
   <222> (1)..(1)
   <223> Phosphorylation at 5'
<400> 242
   gtgtcaagag aaggggcagg gtctatttta aag 33
<210> 243
   <211> 25
   <212> DNA
   <213> Artificial
<220>
   <223> Detection Probe
<220>
   <221> modified_base
   <222> (1)..(1)
   <223> Phosphorylation at 5'
<400> 243
   gtctatctat gctcaccttt ggcag 25
<210> 244
   <211> 29
   <212> DNA
   <213> Artificial
<220>
   <223> Detection Probe
<220>
   <221> modified_base
   <222> (1)..(1)
   <223> Phosphorylation at 5'
<400> 244
   caaatggctt aggcttcaca taaatatcc 29
<210> 245
   <211> 38
   <212> DNA
   <213> Artificial
<220>
   <223> Detection Probe
<220>
   <221> modified_base
   <222> (1)..(1)
   <223> Phosphorylation at 5'
<400> 245
   ttaacatctc tcctttgatg tgtgtgtaag ccttaatg 38
<210> 246
   <211> 35
   <212> DNA
   <213> Artificial
<220>
   <223> Detection Probe
<220>
   <221> modified_base
   <222> (1)..(1)
   <223> Phosphorylation at 5'
<400> 246
   ctatgtcaag tgagctaggt ataatgcatg aagcc 35
<210> 247
   <211> 38
   <212> DNA
   <213> Artificial
<220>
   <223> Detection Probe
<220>
   <221> modified_base
   <222> (1)..(1)
   <223> Phosphorylation at 5'
<400> 247
   ctggaaatca cttagagttg atcttgattg aattagtc 38
<210> 248
   <211> 36
   <212> DNA
   <213> Artificial
<220>
   <223> Detection Probe
<220>
   <221> modified_base
   <222> (1)..(1)
   <223> Phosphorylation at 5'
<400> 248
   gctgccttga cttgttggaa gacattttta catccc 36
<210> 249
   <211> 32
   <212> DNA
   <213> Artificial
<220>
   <223> Detection Probe
<220>
   <221> modified_base
   <222> (1)..(1)
   <223> Phosphorylation at 5'
<400> 249
   cacttcccac atggcttatt ggttctgtgt gc 32
<210> 250
   <211> 26
   <212> DNA
   <213> Artificial
<220>
   <223> Detection Probe
<220>
   <221> modified_base
   <222> (1)..(1)
   <223> Phosphorylation at 5'
<400> 250
   ttagttctgc agtgttcaca ggagcc 26
<210> 251
   <211> 24
   <212> DNA
   <213> Artificial
<220>
   <223> Detection Probe
<220>
   <221> modified_base
   <222> (1)..(1)
   <223> Phosphorylation at 5'
<400> 251
   ggtcccatct tgcttttttc ttcc 24
<210> 252
   <211> 26
   <212> DNA
   <213> Artificial
<220>
   <223> Detection Probe
<220>
   <221> modified_base
   <222> (1)..(1)
   <223> Phosphorylation at 5'
<400> 252
   tacccagaac accatgacat agaagg 26
<210> 253
   <211> 32
   <212> DNA
   <213> Artificial -
<220>
   <223> Detection Probe
<220>
   <221> modified_base
   <222> (1)..(1)
   <223> Phosphorylation at 5'
<400> 253
   gatgcgaagg aagatgactg tggccaggat cc 32
<210> 254
   <211> 41
   <212> DNA
   <213> Artificial
<220>
   <223> Detection Probe
<220>
   <221> modified_base
   <222> (1)..(1)
   <223> Phosphorylation at 5'
<400> 254
   gaagccactt tgatccctgt tttatcgttt gagtttttgt c 41
<210> 255
   <211> 37
   <212> DNA
   <213> Artificial
<220>
   <223> Detection Probe
<220>
   <221> modified_base
   <222> (1)..(1)
   <223> Phosphorylation at 5'
<400> 255
   agcagtggaa gcagcagagt tatgtttgga gcaaaac 37
<210> 256
   <211> 51
   <212> DNA
   <213> Artificial
<220>
   <223> Detection Probe
<220>
   <221> modified_base
   <222> (1)..(1)
   <223> Phosphorylation at 5'
<400> 256
   caagtcagtg tatgtggttg gaaatgctac tgcttctcta ggtaaggagt c 51
<210> 257
   <211> 26
   <212> DNA
   <213> Artificial
<220>
   <223> Detection Probe
<220>
   <221> modified_base
   <222> (1)..(1)
   <223> Phosphorylation at 5'
<400> 257
   agaaggagaa acctgtggaa atgcag 26
<210> 258
   <211> 25
   <212> DNA
   <213> Artificial
<220>
   <223> Detection Probe
<220>
   <221> modified_base
   <222> (1)..(1)
   <223> Phosphorylation at 5'
<400> 258
   ctgggacttt ttaggagtgt ctctg 25
<210> 259
   <211> 31
   <212> DNA
   <213> Artificial
<220>
   <223> Detection Probe
<220>
   <221> modified_base
   <222> (1)..(1)
   <223> Phosphorylation at 5'
<400> 259
   gaaacctcaa aagagaaatc ctgccaaaag c 31
<210> 260
   <211> 30
   <212> DNA
   <213> Artificial
<220>
   <223> Detection Probe
<220>
   <221> modified_base
   <222> (1)..(1)
   <223> Phosphorylation at 5'
<400> 260
   ggattgccat ggaaagcata actgtgtatc 30
<210> 261
   <211> 34
   <212> DNA
   <213> Artificial
<220>
   <223> Detection Probe
<220>
   <221> modified_base
   <222> (1)..(1)
   <223> Phosphorylation at 5'
<400> 261
   gagttatctg gtgacaatat cgatcaaatt aagg 34
<210> 262
   <211> 27
   <212> DNA
   <213> Artificial
<220>
   <223> Detection Probe
<220>
   <221> modified_base
   <222> (1)..(1)
   <223> Phosphorylation at 5'
<400> 262
   agtgaaaggc tcctgtgaaa aagctgc 27
<210> 263
   <211> 25
   <212> DNA
   <213> Artificial
<220>
   <223> Detection Probe
<220>
   <221> modified_base
   <222> (1)..(1)
   <223> Phosphorylation at 5'
<400> 263
   ctagccctgt gagagcttcc tgtgc 25
<210> 264
   <211> 36
   <212> DNA
   <213> Artificial
<220>
   <223> Detection Probe
<220>
   <221> modified_base
   <222> (1)..(1)
   <223> Phosphorylation at 5'
<400> 264
   ctaacagctg tctggggtgg catccgtgga cttcac 36
<210> 265
   <211> 31
   <212> DNA
   <213> Artificial
<220>
   <223> Detection Probe
<220>
   <221> modified_base
   <222> (1)..(1)
   <223> Phosphorylation at 5'
<400> 265
   gctatgggga ccctatccaa atcctcactg g 31
<210> 266
   <211> 25
   <212> DNA
   <213> Artificial
<220>
   <223> Detection Probe
<220>
   <221> modified_base
   <222> (1)..(1)
   <223> Phosphorylation at 5'
<400> 266
   ccaatgggaa aagccttagt tgaac 25
<210> 267
   <211> 34
   <212> DNA
   <213> Artificial
<220>
   <223> Detection Probe
<220>
   <221> modified_base
   <222> (1)..(1)
   <223> Phosphorylation at 5'
<400> 267
   ctgaatgcca gtgatgtgct ctcagccatg gaag 34
<210> 268
   <211> 32
   <212> DNA
   <213> Artificial
<220>
   <223> Detection Probe
<220>
   <221> modified_base
   <222> (1)..(1)
   <223> Phosphorylation at 5'
<400> 268
   gaagtgaaac ctgggctgta accgcctccg ag 32
<210> 269
   <211> 21
   <212> DNA
   <213> Artificial
<220>
   <223> Detection Probe
<220>
   <221> modified_base
   <222> (1)..(1)
   <223> Phosphorylation at 5'
<400> 269
   ccttttgctc gctgtgtggc t 21
<210> 270
   <211> 23
   <212> DNA
   <213> Artificial
<220>
   <223> Detection Probe
<220>
   <221> modified_base
   <222> (1)..(1)
   <223> Phosphorylation at 5'
<400> 270
   tcacgtgagt ctccaagaat ggg 23
<210> 271
   <211> 35
   <212> DNA
   <213> Artificial
<220>
   <223> Detection Probe
<220>
   <221> modified_base
   <222> (1)..(1)
   <223> Phosphorylation at 5'
<400> 271
   ggatgttcct gatgacatac agcctatcac cagcc 35
<210> 272
   <211> 31
   <212> DNA
   <213> Artificial
<220>
   <223> Detection Probe
<220>
   <221> modified_base
   <222> (1)..(1)
   <223> Phosphorylation at 5'
<400> 272
   cccaaggtga agaatcaaag gaagggctaa g 31
<210> 273
   <211> 25
   <212> DNA
   <213> Artificial
<220>
   <223> Detection Probe
<220>
   <221> modified_base
   <222> (1)..(1)
   <223> Phosphorylation at 5'
<400> 273
   gaagaccttc cccaacctcc tggtg 25
<210> 274
   <211> 27
   <212> DNA
   <213> Artificial
<220>
   <223> Detection Probe
<220>
   <221> modified_base
   <222> (1)..(1)
   <223> Phosphorylation at 5'
<400> 274
   tcctgaaggg ctcctgagtg aaaacgg 27
<210> 275
   <211> 24
   <212> DNA
   <213> Artificial
<220>
   <223> Detection Probe
<220>
   <221> modified_base
   <222> (1)..(1)
   <223> Phosphorylation at 5'
<400> 275
   tctgggtcag gaggtggcag agtg 24
<210> 276
   <211> 28
   <212> DNA
   <213> Artificial
<220>
   <223> Detection Probe
<220>
   <221> modified_base
   <222> (1)..(1)
   <223> Phosphorylation at 5'
<400> 276
   cgtggaagcc atcaaagagg ccctgatg 28
<210> 277
   <211> 30
   <212> DNA
   <213> Artificial
<220>
   <223> Detection Probe
<220>
   <221> modified_base
   <222> (1)..(1)
   <223> Phosphorylation at 5'
<400> 277
   cttcctgttt ctatggccct ttccggtgag 30
<210> 278
   <211> 29
   <212> DNA
   <213> Artificial
<220>
   <223> Detection Probe
<220>
   <221> modified_base
   <222> (1)..(1)
   <223> Phosphorylation at 5'
<400> 278
   gatgcagcta agtcaagccc agcttttgg 29
<210> 279
   <211> 30
   <212> DNA
   <213> Artificial
<220>
   <223> Detection Probe
<220>
   <221> modified_base
   <222> (1)..(1)
   <223> Phosphorylation at 5'
<400> 279
   cctacctgga catcgtgcgg gaggtaaagg 30
<210> 280
   <211> 42
   <212> DNA
   <213> Artificial
<220>
   <223> Detection Probe
<220>
   <221> modified_base
   <222> (1)..(1)
   <223> Phosphorylation at 5'
<400> 280
   ctccctctcg ccgtgtacca cgtctctgga gagtttgcca tg 42
<210> 281
   <211> 23
   <212> DNA
   <213> Artificial
<220>
   <223> Detection Probe
<220>
   <221> modified_base
   <222> (1)..(1)
   <223> Phosphorylation at 5'
<400> 281
   ctactacaca ccgcagctgc tgc 23
<210> 282
   <211> 36
   <212> DNA
   <213> Artificial
<220>
   <223> Detection Probe
<220>
   <221> modified_base
   <222> (1)..(1)
   <223> Phosphorylation at 5'
<400> 282
   gtgattgtta ggttgcgcag aggtggtctt atccag 36
<210> 283
   <211> 18
   <212> DNA
   <213> Artificial
<220>
   <223> Tag for Oligo L
<400> 283
   ggccgcggga attcgatt 18
<210> 284
   <211> 18
   <212> DNA
   <213> Artificial
<220>
   <223> Tag for Oligo R
<400> 284
   cactagtgaa ttcgcggc 18
<210> 285
   <211> 21
   <212> DNA
   <213> Artificial
<220>
   <223> Detection Probe as internal control
<400> 285
   gccaccatca gcactatgac c 21
<210> 286
   <211> 23
   <212> DNA
   <213> Artificial
<220>
   <223> Detection Probe as internal control
<220>
   <221> modified_base
   <222> (1)..(1)
   <223> Phosphorylation at 5'
<400> 286
   ccaggacaga tcacatacac tgc 23
<210> 287
   <211> 46
   <212> DNA
   <213> Artificial
<220>
   <223> Detection Probe as internal control
<400> 287
   catttaagac cctgagacta tctgttgaag ctgcctgttt tagcgt 46
<210> 288
   <211> 36
   <212> DNA
   <213> Artificial
<220>
   <223> Detection Probe as internal control
<220>
   <221> modified_base
   <222> (1)..(1)
   <223> Phosphorylation at 5'
<400> 288
   ctgttctgct tgttcacgcc gtttcgtctc aaactc 36
<210> 289
   <211> 50
   <212> DNA
   <213> Artificial
<220>
   <223> Detection Probe as internal control
<400> 289
   ctggctggaa gcttgtttaa ggaactcttc atcagtatgc ttgaaagtgt 50
<210> 290
   <211> 46
   <212> DNA
   <213> Artificial
<220>
   <223> Detection Probe as internal control
<220>
   <221> modified_base
   <222> (1)..(1)
   <223> Phosphorylation at 5'
<400> 290
   ccaaaaggca tctcttcacg gttttctcta cactgattac atctcc 46
<210> 291
   <211> 18
   <212> DNA
   <213> Artificial Sequence
<220>
   <223> Primer R for SEQ ID NO. 284
<400> 291
   gccgcgaatt cactagtg 18